(19) Europäisches Patentamt
European Patent Office
Office européen des brevets

(11) EP 3 043 744 B1

(12) **EUROPEAN PATENT SPECIFICATION**

(45) Date of publication and mention of the grant of the patent:
**01.09.2021 Bulletin 2021/35**

(21) Application number: **14781719.1**

(22) Date of filing: **10.09.2014**

(51) Int Cl.:
***A61F 2/16*** *(2006.01)* ***G02B 1/04*** *(2006.01)*
***G02C 7/04*** *(2006.01)*

(86) International application number:
**PCT/US2014/054946**

(87) International publication number:
**WO 2015/038611 (19.03.2015 Gazette 2015/11)**

(54) **METHODS OF ALTERING OPTICAL POWER OF A LENS**

VERFAHREN ZUR ÄNDERUNG DER OPTISCHEN STÄRKE EINER LINSE

MÉTHODES DE MODIFICATION DE LA PUISSANCE OPTIQUE DE LENTILLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2013 US 201361876845 P**

(43) Date of publication of application:
**20.07.2016 Bulletin 2016/29**

(73) Proprietor: **Battelle Memorial Institute**
**Columbus, OH 43201-2693 (US)**

(72) Inventor: **RISSER, Steven M.**
**Reynoldsburg, Ohio 43068 (US)**

(74) Representative: **Kador & Partner PartG mbB**
**Corneliusstraße 15**
**80469 München (DE)**

(56) References cited:
**EP-A2- 1 632 795** **US-A1- 2005 018 310**
**US-A1- 2011 144 747**

- **KEISUKE AOKI: "Photostabilization of Rhodamine 6G in acetone by [beta]-carotene and t-butyl hydroxyanisole", PHOTOMEDICINE AND PHOTOBIOLOGY, vol. 33, 1 January 2011 (2011-01-01), pages 59-61, XP055164739,**
- **William Reusch: "Photochemistry", , 5 May 2013 (2013-05-05), XP055164826, Retrieved from the Internet: URL:https://www2.chemistry.msu.edu/faculty/reusch/virttxtjml/photchem.htm [retrieved on 2015-01-26]**
- **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 043 744 B1

## Description

## CROSS-REFERENCE TO RELATED APPLICATIONS

This application claims priority to U.S. Provisional Application Serial No. 61/876,845, filed September 12, 2013.

## BACKGROUND

The present disclosure relates to methods and devices that are useful for adjusting the optical power of a lens. Such optical lenses may include lenses in eyewear that are exterior to the eye and ophthalmic lenses that are used in close proximity to the eye.

The eye can suffer from several different defects that affect vision. Common defects include myopia (i.e. nearsightedness) and hyperopia (i.e farsightedness). These types of defects occur when light does not focus directly on the retina, and can be corrected by the use of corrective lenses, such as eyeglasses or contact lenses.

In particular, the lens of the eye is used to focus light on the retina. The lens is usually clear, but can become opaque (i.e. develop a cataract) due to age or certain diseases. The usual treatment in this case is to surgically remove the opaque lens and replace it with an artificial or intraocular lens.

It can be desirable to be able to adjust such lenses, either before they are provided to a user or afterwards. In the case of eyeglasses and/or contact lenses, this permits the economical manufacture of lenses which can then be custom-fitted or adjusted to correct manufacturing defects. Such adjustments can also be useful in correcting misplacement of an intraocular lens during the surgical operation and/or to treat higher order optical aberrations. A common method is to use ultraviolet (UV) activation to induce the change in lens performance, to allow for high spatial resolution of the adjustment (due to the low wavelength of UV). After the lens is adjusted, the lens should not appreciably change in performance over the lifetime of the lens.

U.S. Patent No. 7,134,755 describes a lens that uses ultraviolet light curable monomers in a silicone polymer matrix. The monomers are selectively polymerized using a digital light delivery system to alter the lens power at specific points.

There are two distinct effects that alter the lens optical power in this system. First, the polymerization of the UV curable monomers changes the refractive index of the system from n=1.4144 to n=1.4229, which would increase the optical power of the test lens from 95.1 diopters to 96.7 diopters. This change in the lens power is much smaller than the change in lens power that was reported in the patent, indicating this is not the primary mechanism of index change in this patent.

The second effect, which is responsible for the largest component of the change in lens optical power, is a swelling of the lens in the irradiated region. This swelling effect is illustrated in **FIG. 1.**

In **FIG. 1A,** free monomers (denoted M) are present in a silicone polymer matrix **10.** In **FIG. 1B,** a mask **20** is used to expose only a portion **30** of the lens to UV radiation. The monomers in the region exposed to the UV radiation undergo polymerization, forming polymers P and slightly changing the refractive index. Over time, as seen in **FIG. 1C**, monomers from the un-exposed regions **40, 50** then migrate into the exposed region **30,** causing that region to swell. This change in the lens thickness then leads to a larger change in the optical power. In **FIG. 1D,** after the migration of the monomer is finished, the whole lens is then exposed to UV radiation to freeze the changes.

There are several shortcomings to this method. One is that the primary change in the lens optical power is due to diffusion of monomer, which is a relatively slow process. Another shortcoming is that the dependence on diffusion as the operative effect limits the spatial resolution of the changes in the lens optical power. A third shortcoming is that the increase in lens thickness in the exposed region forces a thickness decrease in adjacent regions, as monomer from the adjacent region diffuses into the exposed region. This change in thickness in the adjacent regions is not easily controllable. Lenses without these shortcomings and others are desirable.

US2011/0144747A1 describes a method of altering the optical power of a lens, comprising: providing a lens comprising a polymer matrix having photobleachable chromophores; and exposing a portion of the lens to radiation, causing photobleaching to occur in the exposed portion of the lens and decreasing the refractive index of the exposed portion of the lens, thereby altering the optical power of the lens.

## BRIEF DESCRIPTION

Disclosed in various embodiments are devices and methods for adjusting the optical power of a lens. The method, of the invention, of altering the optical power of a lens is according to claim 1. Among other benefits, these lenses do not contain free monomers, so there is no change in lens shape due to diffusion of monomers. There is also no need for a second UV radiation exposure of the total lens to "lock-in" the refractive index changes.

Disclosed in some embodiments is a lens comprising: a single polymer matrix having crosslinkable pendant

groups, wherein the polymer matrix increases in volume when crosslinked; and wherein substantially no free monomers are present therein.

The lens may further comprise a UV absorbing layer on at least one surface of the lens.

The pendant group may be 3,9-divinyl-2,4,8,10-tetraoxy-spiro[5.5]undecane.

Disclosed in other embodiments is a lens comprising a polymer matrix including photobleachable chromophores.

The photobleachable chromophores may be dispersed within the polymer matrix, or be present as pendant groups on the polymer matrix.

At least one chromophore may comprise a reactive site which can crosslink with a reactive site on the polymer matrix.

The photobleachable chromophores may comprise chromophores containing a malononitrile moiety, such as those of Formula (I) or Formula (II):

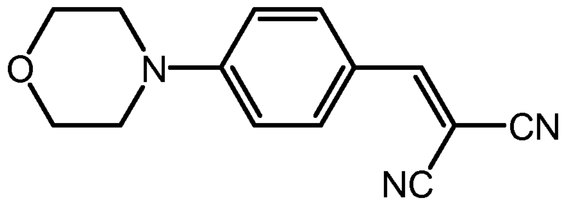

**Formula (I)**

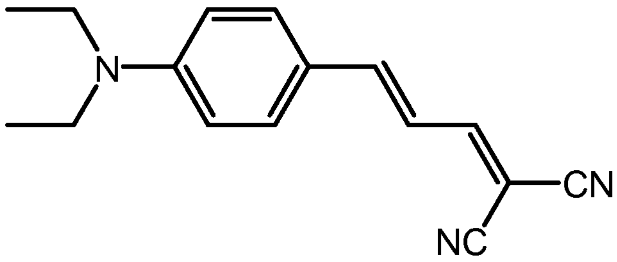

**Formula (II)**.

Alternatively, the photobleachable chromophores may comprise stilbene chromophores of Formula (III):

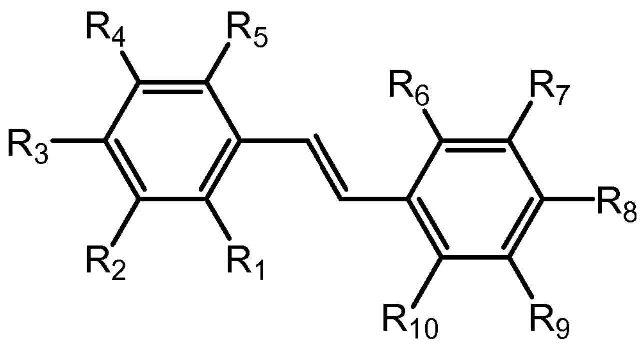

**Formula (III)**

where $R_1$-$R_{10}$ are independently selected from hydrogen, alkyl, substituted alkyl, aryl, substituted aryl, -COOH, and $-NO_2$.

Alternatively, the photobleachable chromophores may comprise azobenzene chromophores of Formula (IV):

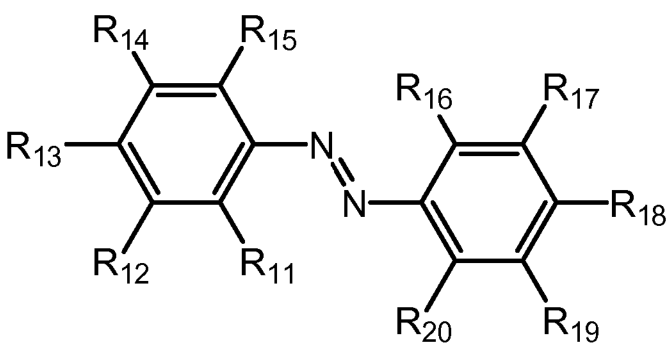

**Formula (IV)**

where $R_{10}$-$R_{20}$ are independently selected from hydrogen, alkyl, substituted alkyl, aryl, substituted aryl, -COOH, $-NO_2$, halogen, amino, and substituted amino.

In other embodiments, at least one chromophore must absorb more than one photon for photobleaching of the chromophore to occur.

Disclosed in still other embodiments is a method of altering the optical power of a lens, comprising: providing a lens comprising: a single polymer matrix having crosslinkable pendant groups, wherein the polymer matrix increases in volume when crosslinked; and wherein the lens is devoid of free monomers; and exposing a portion of the lens to radiation, causing crosslinking to occur in the exposed portion of the lens and changing the refractive index of the exposed portion of the lens, thereby altering the optical power of the lens.

The exposed portion of the lens may be in the center of the lens. The radiation to which the lens is exposed may have a wavelength of from about 200 nm to about 600 nm.

In other embodiments is disclosed a method of altering the optical power of a lens, comprising: providing a lens comprising a polymer matrix having photobleachable chromophores; and exposing a portion of the lens to radiation, causing photobleaching to occur in the exposed portion of the lens and changing the refractive index of the exposed portion of the lens, thereby altering the optical power of the lens.

These and other non-limiting aspects and/or objects of the disclosure are more particularly described below.

## BRIEF DESCRIPTION OF THE DRAWINGS

The following is a brief description of the drawings, which are presented for the purposes of illustrating the disclosure set forth herein and not for the purposes of limiting the same.

**FIGS. 1A-1D** are illustrations of a conventional method for adjusting lens optical power.

**FIG. 2** is a graph showing a normalized change in lens optical power as a function of the refractive index of the lens in both air and water.

**FIGS. 3A-3B** are illustrations of one method of the present disclosure for altering the optical power of a lens.

**FIG. 4** is a graph showing the change in the refractive index as a function of the change in the volume of a polymer used to make the lens.

**FIG. 5** is a graph showing the change in the lens optical power as a function of the change in the volume of a polymer used to make the lens.

**FIGS. 6A-6B** are illustrations of another method of the present disclosure for altering the optical power of a lens.

**FIG. 7** is a graph of the solar spectrum, showing the amount of energy at each wavelength.

**FIG. 8** is a graph showing the photon flux and the chromophore lifetime as a function of the wavelength.

**FIGS. 9A-9C** are three figures describing bleaching via two-photon absorption by a chromophore.

**FIG. 10** is a cross-sectional view of an exemplary embodiment of a lens of the present disclosure.

**FIG. 11** is an idealized transmission spectrum for a UV radiation absorbing layer of the present disclosure.

**FIG. 12** is a graph showing the refractive index of a lens as a function of the amount of time the lens was exposed to UV radiation.

**FIG. 13** is a graph showing the transmission spectrum of a contact lens before application of a chromophore, before bleaching, and after bleaching.

## DETAILED DESCRIPTION

A more complete understanding of the processes and apparatuses disclosed herein can be obtained by reference to the accompanying drawings. These figures are merely schematic representations based on convenience and the ease of demonstrating the existing art and/or the present development, and are, therefore, not intended to indicate relative size and dimensions of the assemblies or components thereof.

Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

The modifier "about" used in connection with a quantity is inclusive of the stated value and has the meaning dictated by the context (for example, it includes at least the degree of error associated with the measurement of the particular quantity). When used with a specific value, it should also be considered as disclosing that value. For example, the term "about 2" also discloses the value "2" and the term "from about 2 to about 4" also discloses the range "from 2 to 4."

References to ultraviolet or UV radiation should be understood as referring to the portion of the light spectrum having wavelengths between about 400 nm and about 10 nm.

The "refractive index" of a medium is the ratio of the speed of light in a vacuum to the speed of light in the medium. For example, the refractive index of a material in which light travels at two-thirds the speed of light in a vacuum is (1 / (2/3)) = 1.5.

The term "chromophore" refers to a chemical moiety or molecule that has a substantial amount of aromaticity or conjugation. This aromaticity or conjugation acts to increase the absorption strength of the molecule and to push the absorption maximum to longer wavelengths than is typical for molecules that only have sigma bonds. In many cases this chromophore will act to impart color to a material. As defined here, the chromophore does not need to absorb in the visible (i.e. does not need to be colored), but can have its absorption maximum in the UV. Alternately, the chromophore could have absorption maximum in the near-IR, with no significant absorption in the visible wavelength range. The chromophore has a refractive index larger than that of the base polymer.

Non-limiting examples of chromophores which act to impart color to a material include C.I. Solvent Blue 101; C.I. Reactive Blue 246; C.I. Pigment Violet 23; C.I. Vat Orange 1; C.I. Vat Brown 1; C.I. Vat Yellow 3; C.I. Vat Blue 6; C.I. Vat Green 1; C.I. Solvent Yellow 18; C.I. Vat Orange 5; C.I. Pigment Green 7; D&C Green No. 6; D&C Red No. 17; D&C Yellow No. 10; C.I. Reactive Black 5; C.I. Reactive Blue 21; C.I. Reactive Orange 78; C.I. Reactive Yellow 15; C.I. Reactive Blue 19; C.I. Reactive Blue 4; C.I. Reactive Red 11; C.I. Reactive Yellow 86; C.I. Reactive Blue 163; and C.I. Reactive Red 180.

Additional molecules which could act as a chromophore for this disclosure, but will not impart color to a material, include derivatives of oxanilides, quinones, benzophenones, benzotriazoles, hydroxyphenyltriazines, and other polyaromatic molecules. Other examples can be found in Dexter, "UV Stabilizers", Kirk-Othmer Encyclopedia of Chemical Technology 23: 615-627 (3d. ed. 1983), U.S. Patent No. 6,244,707, and U.S. Patent No. 4,719,248.

Other molecules which can act as chromophores for this disclosure include unsaturated molecules found in nature, such as riboflavin, lutein, β-carotene, cryptoxanthin, zeaxanthin, or Vitamin A, as examples.

The term "photobleaching" refers to a change in the chromophore induced by photochemical means. Exemplary changes may be the cleavage of the chromophore into two or more fragments, or a change in the bond order of one or more covalent bonds in the chromophore, or a rearrangement of the bonds, such as a transition from a trans-bonding pattern to a cis-bonding pattern. Alternately, the change could be the cleavage of a bond such that the chromophore is no longer covalently bound to the polymer matrix, allowing the chromophore to be removed during wash steps.

The term "optical lens" is used herein to refer to a device through which vision can be modified or corrected, or through which the eye can be cosmetically enhanced (e.g. by changing the color of the iris) without impeding vision. Non-limiting examples of optical lenses include eyewear and ophthalmic lenses. The term "ophthalmic lenses" refers to those devices that contact the eye. Examples of ophthalmic lenses include contact lenses and intraocular lenses. Examples of eyewear include glasses, goggles, full face respirators, welding masks, splash shields, and helmet visors.

The optical power of a simple lens is given by the following Equation 1:

$$\frac{1}{f} = (n - n_0)\left[\frac{1}{R_1} - \frac{1}{R_2} + \frac{(n-n_0)d}{nR_1R_2}\right] \qquad (1)$$

where 1/f is the optical power of the lens (measured in diopters or $m^{-1}$), $n$ is the refractive index of the lens material, $n_0$ is the refractive index of the surrounding medium, $R_1$ and $R_2$ are the two radii of curvature of the lens, and d is the thickness of the lens.

The importance of change in the refractive index is shown in **FIG. 2**, which is a graph showing the normalized change in lens optical power as a function of the refractive index for a lens placed both in water and air (normalized by the lens power at n=1.5). The calculations were performed using $R_1$=0.00185 m, $R_2$=0.00255 m, d=300 μm, $n_0$ for water=1.3374, and $n_0$ for air=1.0000.

In some methods of the present disclosure, crosslinking is used to change the refractive index of the lens. The lens thickness may either slightly shrink or increase, but the lens curvature is not appreciably altered. The primary change in lens optical power comes from the change in refractive index, not from the change in lens thickness or curvature. This is illustrated in **FIG. 3.** In **FIG. 3A,** the lens **100** contains a polymer matrix (denoted as P) having crosslinkable pendant groups (denoted as X). A mask **105** is used to expose only a portion **110** of the lens to UV or other radiation. As seen in **FIG. 3B**, crosslinking occurs in the exposed portion **110** of the lens, changing the refractive index of the exposed portion.

A lens which is useful in this method may comprise a conventional polymer capable of behaving as a hydrogel, i.e. which can swell upon contact with water. Typically, crosslinking a conventional polymer decreases the volume of the polymer, similar to the decrease in volume upon polymerization (i.e. a decrease in thickness occurs). This reduction in volume leads to an increased refractive index. However, there are monomers, such as 3,9-divinyl-2,4,8,10-tetraoxyspiro[5.5]undecane (shown below), which expand under photopolymerization.

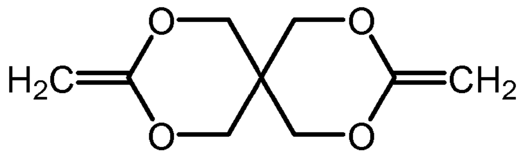

3,9-divinyl-2,4,8,10-tetraoxy-spiro[5.5]undecane

Including similar functional groups as reactive sidechains or pendant groups in the polymer may lead to an increase in volume upon crosslinking. After crosslinking these functional groups, the regions where the polymer volume has increased will have decreased refractive index, while areas where the polymer volume decreases will have increased refractive index. Put another way, the crosslinked regions of the lens have increased refractive index.

Alternatively, crosslinking a hydrogel controls the degree to which it can swell in the presence of water, preventing an increase in volume. After crosslinking, those regions where the hydrogel has been crosslinked will have an increased refractive index compared to the regions where the hydrogel has not been crosslinked.

Because the change in lens optical power of such lenses does not rely on diffusion of free monomers, the change in lens power can be monitored in real time. Millan in Polymer 46 (2005), pp. 5556-5568, discloses a crosslinked polystyrene which increases refractive index and thickness after crosslinking.

One approach to approximate the role of crosslinking on the optical properties of polymers is to use the Lorentz-Lorenz formalism, which expresses the refractive index in terms of a molar refractivity $R_{LL}$ and molar volume V, as in Equation 2:

$$n = \left[\frac{1+2R_{LL}/V}{1-2R_{LL}/V}\right]^{1/2} \quad (2)$$

The effect of crosslinking is then treated as solely altering the molar volume, without changing the molar refractivity. **FIG. 4** shows the change in the refractive index associated with a change in the volume using Equation 2. **FIG. 5** shows the change in the lens power as a function of the change in volume. The calculations were performed using polymethyl methacrylate (PMMA) as the model compound, with MW=100.117, $R_{LL}$=24.754, and starting volume of V=865 (see van Krevelen, Properties of Polymers, 1976). The volume was systematically decreased and the refractive index was calculated. The lens power calculations were again performed using $R_1$=0.00185 m, $R_2$=0.00255 m, d=300 $\mu$m, $n_0$ for water=1.3374, and $n_0$ for air=1.0000. The calculated refractive index started at n=1.48415, and ended at n=1.50129 ($\Delta$n=0.01714, 1.15%).

**FIG. 5** shows that a change of up to about 10% in lens optical power can occur for a change in volume of less than about 3%, corresponding to a thickness change in the lens of less than about 1%. The calculations are fairly insensitive to whether the volume change is modeled as just corresponding to a thickness change, or is modeled as changing in all 3 dimensions equally.

In embodiments, the lens suitable for practicing this method may comprise a single polymer matrix containing crosslinkable pendant groups, wherein the polymer matrix increases in volume when crosslinked. The lens does not contain free monomers that diffuse between regions to increase the volume. Rather, the increase in volume is due to diffusion of water into the exposed (i.e. crosslinked) portion of the lens.

In embodiments, a method for altering the optical power of a lens comprises providing a lens comprising a single polymer matrix having crosslinkable pendant groups, wherein the polymer matrix increases in volume when crosslinked. The lens is devoid of, i.e. does not contain, free monomers. A portion of the lens is exposed to radiation, such as ultraviolet radiation. This causes crosslinking to occur in the exposed portion of the lens and changes the refractive index of the exposed portion. The refractive index may increase or decrease, and decreases in particular embodiments. In particular embodiments, the exposed portion is in the center of the lens.

In other methods of the present disclosure, ultraviolet (UV) radiation is used to photobleach the lens material. Certain aromatic groups, such as naphthalene, can degrade under UV radiation exposure. This leads to a decrease in the refractive index in these exposed regions, without any change in lens thickness. **FIG. 6** is a schematic of the photobleaching process. In **FIG. 6A,** the lens **100** contains a polymer matrix (denoted as P) having photobleachable chromophores (denoted as C). A mask **105** is used to expose only a portion **110** of the lens to UV or other radiation. As seen in **FIG. 6B,** the chromophores in the exposed portion **110** of the lens are bleached (denoted as B), lowering the refractive index of the exposed portion compared to the unexposed portions **120, 130.** Photobleaching has exceptional spatial resolution, commonly on the order of a few microns. There is extensive literature on the design of chromophores to photobleach and on the design of optical materials with enhanced photostability.

The photobleaching of a material can be described using Equation 3:

$$\frac{B}{\sigma} = \tau n \qquad (3)$$

where B is the probability of the degradation event happening, $\sigma$ is the cross section, n is the photon flux, and $\tau$ is the lifetime of the chromophore. B/ $\sigma$ is often referred to as the photostability Figure-of-Merit (FOM). B/ $\sigma$ has strong energy dependence and also strong dependence on the maximum absorption wavelength ($\lambda_{max}$) of the chromophore.

The energy dependence can be approximated with Equation 4:

$$\log\left[\frac{B}{\sigma}\right] = 24 + 5.0 \times (E_{max} - E) \qquad (4)$$

where $E_{max}$ is the energy of the chromophore maximum absorption wavelength.

The next step in the chromophore lifetime calculation is determination of the maximum and average photon flux the lens will be exposed to. The solar spectrum has the form of **FIG. 7,** and is approximated by the solid line. Long wavelength radiation will be ignored in the determination, as it will have no effect on the photodegradation.

After conversion of the solar spectrum into photon flux, and using the energy dependent FOM expression assuming chromophore absorption maximum of 325 nm, the plot of the chromophore lifetime as a function of the energy in the solar spectrum can be obtained, and is shown as **FIG. 8.** The solid line is the chromophore lifetime, while the dotted line is the photon flux.

The total chromophore lifetime is obtained from the summation of the inverse lifetimes (the total degradation rate is the sum of the individual degradation rates). In this example, the total chromophore lifetime is calculated to be about $2.1 \times 10^5$ seconds. Notably, there is a rapid increase in lifetime as the wavelength increases. Much less than 1% of the photodegradation in this example arises from wavelengths longer than about 400 nm.

The previous calculation for the total chromophore lifetime assumes that the user stares directly into the noontime sun for the entire lens lifetime, which overestimates the total photon exposure during the lens lifetime. Using an ambient light level of 32,000 lux (average noontime level) for 9 hours, 9 hours of 400 lux (ambient office lighting) and 6 hours of sleep per day, the photon flux is calculated to be overestimated by a factor of 30, leading to a predicted lifetime for the lens of about $6.2 \times 10^6$ seconds. This lifetime is still shorter than desired (~$2 \times 10^9$ seconds is desired), but literature precedent shows straight-forward methods to increase the lifetime by more than the three orders of magnitude needed.

The final issue is the amount of optical power available for the photobleaching. Based on 2006 Trans. Am. Ophthalmol. Soc. p. 29, where a power of 12 $mW/cm^2$ was used for 120 seconds ($\lambda$=365nm), the photon flux is $2.2 \times 10^{20}/(m^2 \cdot sec)$, and the total photon exposure is $2.7 \times 10^{22}/m^2$. Staring into the sun, the photon flux below 400 nm is $7.6 \times 10^{19}/(m^2 \cdot sec)$, the ambient noontime flux below 400 nm is approximately $2.5 \times 10^{19}/(m^2 \cdot sec)$, and the average flux is $2.5 \times 10^{18}/(m^2 \cdot sec)$.

In embodiments, a lens suitable for practicing this method comprises a polymer matrix containing photobleachable chromophores. The chromophores may be present as compounds dispersed within the polymer matrix or as pendant groups on the polymer matrix. The chromophores may contain a reactive site which can react with a reactive site on the polymer matrix to allow crosslinking.

In particular embodiments, the chromophore contains a malononitrile moiety. Exemplary chromophores include those of Formulas (I) and (II), which are also known as VC60 and EC24, respectively:

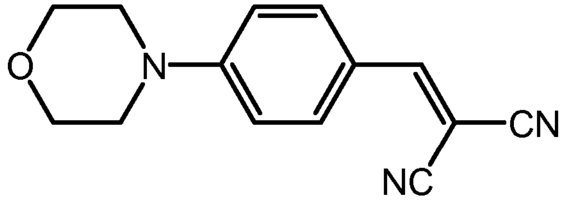

**Formula (I)**

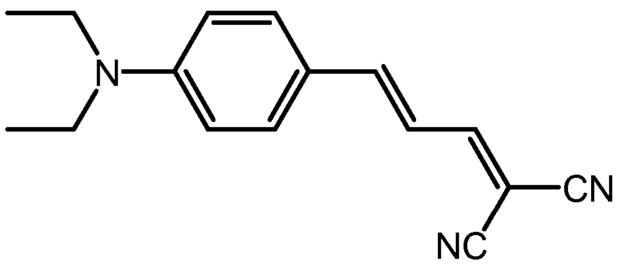

**Formula (II)**

Formula (I) may also be called 4-morpholinobenzylidene malononitrile. Formula (II) may also be called 2-[3-(4-N,N-diethylanilino)propenylidene] malononitrile.

In other embodiments, the chromophore is a stilbene compound of Formula (III):

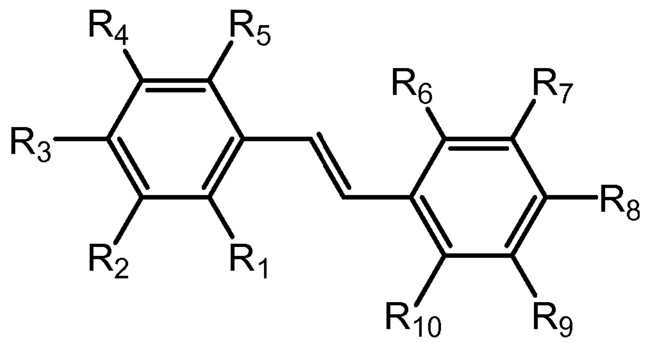

**Formula (III)**

where $R_1$-$R_{10}$ are independently selected from hydrogen, alkyl, substituted alkyl, aryl, substituted aryl, -COOH, and -$NO_2$.

The term "alkyl" as used herein refers to a radical which is composed entirely of carbon atoms and hydrogen atoms which is fully saturated. The alkyl radical may be linear, branched, or cyclic. Linear alkyl radicals generally have the formula -$C_nH_{2n+1}$.

The term "aryl" refers to an aromatic radical composed of carbon atoms and hydrogen atoms. When aryl is described in connection with a numerical range of carbon atoms, it should not be construed as including substituted aromatic radicals. For example, the phrase "aryl containing from 6 to 10 carbon atoms" should be construed as referring to a phenyl group (6 carbon atoms) or a naphthyl group (10 carbon atoms) only, and should not be construed as including a methylphenyl group (7 carbon atoms). The term "heteroaryl" refers to an aryl radical which is not composed of entirely carbon atoms and hydrogen atoms, but rather also includes one or more heteroatoms. The carbon atoms and the heteroatoms are present in a cyclic ring or backbone of the radical. The heteroatoms are selected from O, S, and N. Exemplary heteroaryl radicals include thienyl and pyridyl.

The term "substituted" refers to at least one hydrogen atom on the named radical being substituted with another functional group selected from halogen, -CN, - $NO_2$, -COOH, and -$SO_3H$. An exemplary substituted alkyl group is a perhaloalkyl group, wherein one or more hydrogen atoms in an alkyl group are replaced with halogen atoms, such as fluorine, chlorine, iodine, and bromine. Besides the aforementioned functional groups, an alkyl group may also be substituted with an aryl group. An aryl group may also be substituted with alkyl. Exemplary substituted aryl groups include methylphenyl and trifluoromethylphenyl.

Generally, the substituents $R_1$-$R_{10}$ are selected to enhance other properties of the chromophore. For example, $R_1$, $R_5$, $R_6$, or $R_{10}$ could be selected to be a crosslinkable group, such as a carboxylic acid. The substituents may also be selected as to control the absorption maximum and/or the refractive index of the chromophore, such as trifluoromethyl (to lower the refractive index), or a nitro group (to redshift the absorption maximum). The substituents may also be selected to enhance the photostability of the chromophore. For example, inclusion of a bulky group at the 2 or 2' position, such as phenyl, inhibits trans-cis isomerization.

In other embodiments, the chromophore is an azobenzene compound of Formula (IV):

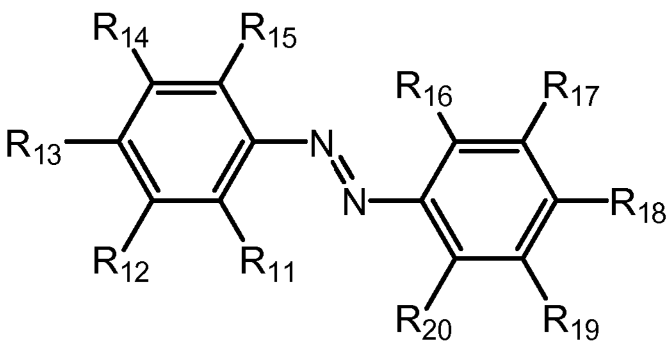

**Formula (IV)**

where $R_{10}$-$R_{20}$ are independently selected from hydrogen, alkyl, substituted alkyl, aryl, substituted aryl, -COOH, -$NO_2$,

halogen, amino, and substituted amino. Generally, the substituents $R_{10}$-$R_{20}$ are selected to enhance other properties of the chromophore.

The term "amino" refers to $-NH_2$.

In still other embodiments, the chromophore must absorb more than one photon for bleaching to occur. **FIG. 9** provides an explanation. As illustrated in **FIG. 9A,** the chromophore molecule has three energy levels, which include the ground state G, the first excited state B which can be accessed from the ground state by the absorption of a single photon $\omega_{max}$, and the second excited state A which cannot be accessed from the ground state by a single photon absorption. Typically, there is an allowed transition between states B and A. The second excited state A is the state where the chromophore bleaches. Analysis of the one and two photon absorption spectra for simple chromophores indicates that the energy of state B relative to the ground state is approximately 0.85 of the energy of state A relative to the ground state (Marius Albota, Science 281, p. 1653 (1998)). **FIG. 9B** shows the energy spectrum for a standard two-photon absorption. In this process, two photons $\omega$ of the same wavelength are absorbed, when the energy of a single photon is too small to be directly absorbed. In this case, the absorption rate depends on the square of the optical intensity. It is also possible to have a two photon absorption where the two photons are of different frequencies. This is shown in **FIG. 9C,** where two distinct photons w1 and $\omega2$ are absorbed, even though neither photon alone has sufficient energy to excite the molecule to even the first excited state B. In this case, the absorption intensity is proportional to the product of the intensity of each wavelength.

In embodiments, a method for altering the optical power of a lens comprises providing a lens comprising a polymer matrix containing photobleachable chromophores. A portion of the lens is exposed to radiation, such as ultraviolet radiation. This causes photobleaching to occur in the exposed portion of the lens and changes the refractive index of the exposed portion. The refractive index may increase or decrease, and decreases in specific embodiments. In particular embodiments, the exposed portion is in the center of the lens.

After the optical power of the lens is altered, the lens must be stabilized to prevent further undesired changes. Previous lenses which include free monomer(s) typically used partial polymerization, allowed the free monomer(s) to diffuse, then did a complete polymerization to preclude any further change in the shape of the lens or the refractive index. The present disclosure contemplates at least three methods of stabilization.

First, a UV radiation absorbing layer may be laid over at least one surface of the lens. The UV radiation absorbing layer ideally almost completely absorbs short wavelength photons at low UV intensity, but passes most photons at high UV intensity. An exemplary lens is shown in **FIG. 10.** Here, the lens **200** comprises a polymer matrix **210** and UV radiation absorbing layers **220, 230** on each surface **212, 214** of the polymer matrix. An idealized transmission spectrum at the bleaching wavelength and longer is shown in **FIG. 11.** At wavelengths shorter than the bleaching wavelength, the UV absorption layer completely absorbs photons. At the bleaching wavelength and longer, however, the photon absorption depends on the incident flux. At low levels of incident UV intensity, i.e. that of natural illumination, the transmitted flux (i.e. the number of photons passing through the UV absorption layer) is low or zero. At higher levels of incident UV intensity, however, i.e. applied illumination, the transmitted flux increases. This difference allows the lens to be adjusted after implantation by the application of artificial radiation, then prevent further adjustment during natural use. This UV absorbing layer can be used with both types of lenses described above.

The second stabilization method involves crosslinking the chromophore to the polymer matrix, through for example the 2' position. The chromophore can be attached to the polymer matrix as a pendant group or sidechain with a reactive site or group on the chromophore, and a corresponding reaction site or group elsewhere on the polymer matrix. After fabrication, the lens can be stored at a temperature below the Tg of the polymer, greatly slowing the crosslinking reaction. After implantation, the chromophore will slowly crosslink with the polymer matrix, greatly enhancing its photostability. The rate of this crosslinking reaction can be controlled by altering the functionality of the reactive groups, allowing sufficient time for the lens to be adjusted. If the crosslinking occurs through a condensation reaction, water will be the only byproduct. After crosslinking, there will be a further reduction in the rate at which the isomerization can occur, further enhancing photostability.

Crosslinking the chromophore through its 2' position is significant because of the degradation mechanism of, for example, stilbene chromophores. The primary degradation pathway of stilbene chromophores is through oxidation of the central double bond after a trans-cis isomerization. Thus, blocking groups have also shown an increase in the chromophore stability. As shown in the following diagram, the unsubstituted stilbene can undergo trans-cis isomerization, while the substituted stilbene is sterically hindered. By hindering isomerization, stability is increased.

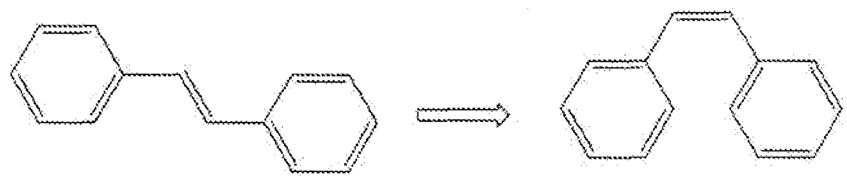

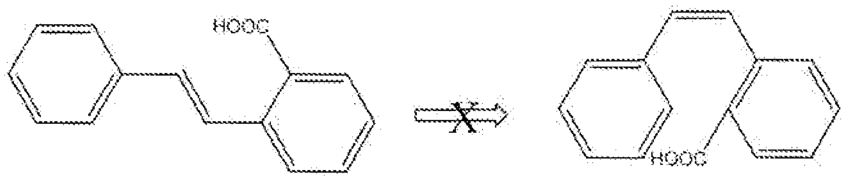

Finally, the third stabilization method uses a chromophore which bleaches under specific conditions. In particular, a chromophore which requires the absorption of more than one photon to bleach is used. The bleaching process is slow under low-level illumination, but may still occur, particularly during daytime outside exposure. However, judicious selection of the excitation wavelengths of the chromophore can slow this process even further. Referring back to **FIG. 7,** certain wavelengths are filtered from the solar spectrum, due to the presence of specific compounds in the atmosphere. Design of the chromophore so that one of the two wavelengths needed to cause bleaching corresponds to one of the filtered wavelengths will lead to a lens with enhanced stability, as there will be no high-intensity natural radiation at that wavelength to initiate the photodegradation.

For example, if we assume a chromophore with an absorption maximum of 400 nm, the two-photon absorption would then occur at about 340 nm. If one of the wavelengths is 1300 nm (which is strongly absorbed in the atmosphere), we can the calculate that the other wavelength needed would be 460 nm. Thus a combination of a 1300 nm photon and a 460 nm photon could be absorbed, even though neither photon will be absorbed individually. Because the 1300 nm photon is not strongly present in natural outdoor illumination, absorption and continued photobleaching would not occur.

The photostability of chromophores may also be enhanced in other ways. The chromophores may be attached to the polymer matrix as a polymer side chain or pendant group. Chromophores could be crosslinked to other functional groups on the polymer backbone or sidechains, reducing the conformational movement often needed as part of the photobleaching process. The absorption maxiumum wavelength could be blue-shifted. The functional groups of the chromophore could be changed to inhibit rotational motion around specific bonds, or block specific photodegradation pathways. For example, inclusion of a trifluoromethyl group at the 2 or 2' position of an azobenzene chromophore can reduce the rate at which photobleaching occurs. Finally, the local environment of the chromophore could be changed, e.g. by changing the local pH.

Several other methods of altering the optical power of a lens are also disclosed. These methods relate to lenses that use photobleachable chromophores and a polymer matrix. Generally, exposure to radiation is used to cause bleaching with different variations.

In the method of the invention the lens has a first optical power and comprises a polymer matrix having photobleachable chromophores. A radical scavenger is also dispersed in the polymer matrix. In this regard, the photodegradation of chromophores commonly occurs through generation of a radical. When a radical scavenger is incorporated into the lens at a relatively low level, any radicals that are generated under standard illumination are absorbed by the radical scavenger instead of bleaching the chromophore. However, when the lens is intentionally exposed to radiation, the radical scavenger is unable to scavenge all the radicals created during such a photobleaching process. Thus, it is contemplated that when a portion of the lens is exposed to radiation, photobleaching will occur in the exposed portion of the lens. In turn, the bleaching of the chromophores changes the refractive index of the exposed portion and the optical power of the lens is thus altered to a second optical power which is different from the first optical power.

In some embodiments, two or more different radical scavengers may be added to the lens. The radical scavenger(s) may be selected from hindered phenols, secondary aromatic amines, hydroquinones, benzoquinones, piperazines, polybutadienes, and mercaptans. The radical scavenger(s) may be added in an amount of from about 0.01 to about 10 wt% of the lens. The radical scavenger(s) generally are dispersed homogeneously through the polymer matrix.

In some embodiments, the hindered phenol is an alkyl substituted phenol of Formula (V):

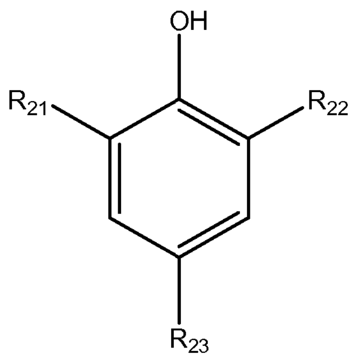

**Formula (V)**

wherein $R_{21}$, $R_{22}$, and $R_{23}$ are independently selected from the group consisting of hydrogen, alkyl, and substituted alkyl; and wherein at least one of $R_{21}$ and $R_{22}$ is not hydrogen. In some embodiments, $R_{21}$ and $R_{22}$ are not hydrogen.

In some embodiments, the secondary aromatic amine is a compound of Formula (VI):

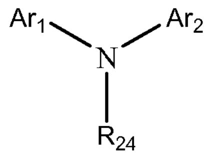

**Formula (VI)**

wherein $Ar_1$ and $Ar_2$ represent the same or different aryl, or substituted aryl; and wherein $R_{24}$ is selected from hydrogen, alkyl, substituted alkyl, -COOH, $-NO_2$, halogen, amino, and substituted amino.

In some embodiments, the hydroquinone is a compound of Formula (VII):

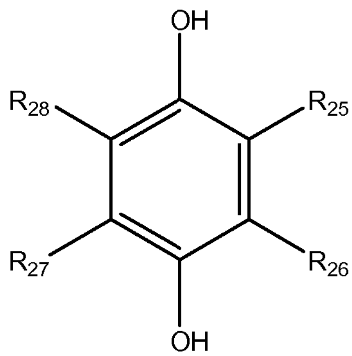

**Formula (VII)**

wherein $R_{25}$-$R_{28}$ are independently selected from hydrogen, alkyl, substituted alkyl, aryl, substituted aryl, -COOH, $-NO_2$, halogen, amino, and substituted amino. In some embodiments, $R_{25}$-$R_{28}$ are hydrogen.

In some embodiments, the benzoquinone is a para-benzoquinone compound of Formula (VIII) or an ortho-benzoquinone of Formula (IX):

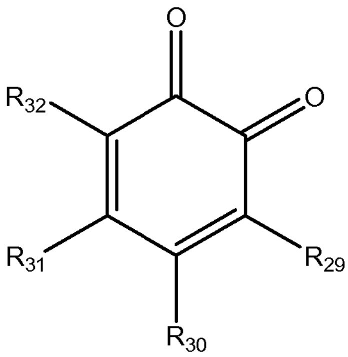

**Formula (XIII)**

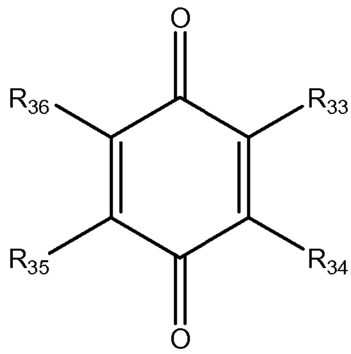

**Formula (IX)**

wherein $R_{29}$-$R_{36}$ are independently selected from hydrogen, alkyl, substituted alkyl, aryl, substituted aryl, -COOH, -$NO_2$, halogen, amino, and substituted amino. In some embodiments, $R_{29}$-$R_{32}$ and/or $R_{33}$-$R_{36}$ are hydrogen. Both para- and ortho- isomers may be present in the same composition.

In some embodiments, the piperazine is a compound of Formula (X):

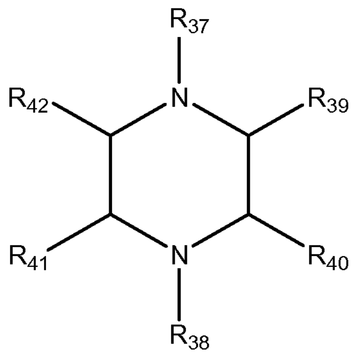

**Formula (X)**

wherein $R_{37}$-$R_{42}$ are independently selected from hydrogen, alkyl, substituted alkyl, aryl, substituted aryl, -COOH, -$NO_2$, halogen, amino, and substituted amino. In some embodiments, $R_{37}$ and $R_{38}$ are both hydrogen.

In some embodiments, the polybutadiene is a cis polymer of Formula (XI) or a trans polymer of Formula (XII):

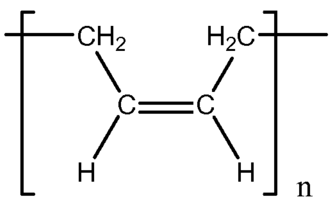

**Formula (XI)**

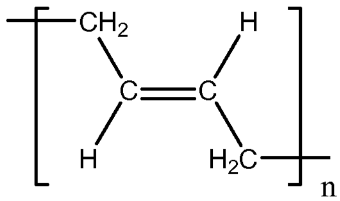

**Formula (XII)**

wherein n represents the number of repeating units, and between 10 and 5,000. In some embodiments, both cis-polybutadiene and trans-polybutadiene are present in the composition.

In some embodiments, the mercaptan is a compound of Formula (XII):

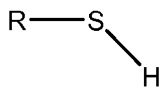

**Formula (XIII)**

wherein R is a carbon-containing moiety. In some embodiments, R is selected from the group consisting of alkyl, substituted alkyl, aryl, substituted aryl, and -COOH.

In other methods, the lens has a first optical power and comprises a polymer matrix having photobleachable chromophores. A sensitizer is added to the lens. Many compounds exist which can act as a sensitizer or catalyst that causes the chromophore to be photodegraded at a higher rate. Upon exposing a portion of the lens to radiation, the sensitizer acts as a catalyst to cause photobleaching to occur in the exposed portion of the lens. This changes the refractive index of the exposed portion of the lens and altering the optical power of the lens.

In some embodiments, two or more different sensitizers may be added to the lens. The sensitizer(s) may be selected from aromatic or aliphatic photoinitiators, or aromatic ketones such as benzophenones and thioxanthones. Exemplary sensitizers include Irgacure® molecules available from Ciba, such as 1-hydroxy-cyclohexyl-phenylketone (Irgacure® 184); 2-hydroxy-1-{4-[4-(2-hydroxy-2-methyl-propionyl)-benzyl]-phenyl}-2-methyl-propan-1-one (Irgacure® 127); 2-hydroxy-1-[4-(2-hydroxyethoxy)phenyl]-2-methyl-propanone (Irgacure® 2959); bis(2,4,6-trimethylbenzoyl)-phenylphosphineoxide (Irgacure® 819); 2-methyl-1-[4-(methylthio)phenyl]-2-morpholinopropan-1-one (Irgacure® 907); 2-benzyl-2-dimethylamino-1-(4-morpholinophenyl)-butan-1-one (Irgacure® 369); 2-dimethylamino-2-(4-methylbenzyl)-1-(4-morpholin-4-yl-phenyl)-butan-1-one (Irgacure® 379); and 2,2-dimethoxy-1,2-diphneylethan-1-one (Irgacure® 651).

In some embodiments, the benzophenone is a compound of Formula (XIV):

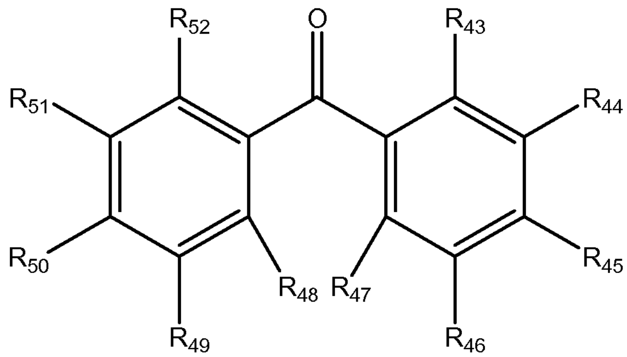

**Formula (XIV)**

wherein $R_{43}$-$R_{52}$ are independently selected from hydrogen, alkyl, substituted alkyl, aryl, substituted aryl, -COOH, $-NO_2$, halogen, amino, and substituted amino. In some embodiments, $R_{43}$-$R_{52}$ are hydrogen.

In some embodiments the thioxanthone is a compound of Formula (XV):

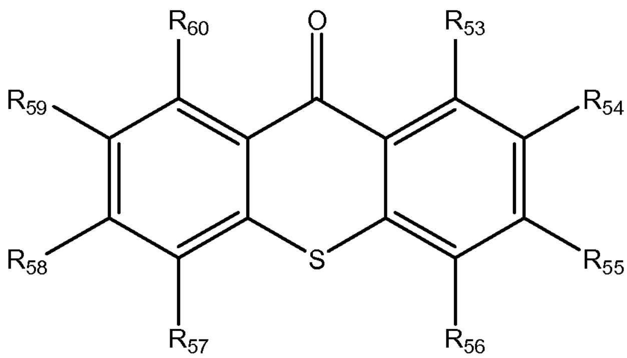

**Formula (XV)**

wherein $R_{53}$-$R_{60}$ are independently selected from hydrogen, alkyl, substituted alkyl, aryl, substituted aryl, -COOH, $-NO_2$, halogen, amino, and substituted amino. In some embodiments, $R_{53}$-$R_{60}$ are hydrogen.

The sensitizer(s) may be added to the lens in an amount of from about 0.01 to about 10 wt% before the exposing. In some embodiments, the sensitizer is added to the lens via injection. The sensitizer(s) may be added from about 24 hours before exposure to immediately before the exposure of the lens to radiation. More specifically, the sensitizer can be locally added to only the portion of the lens that is to be exposed to radiation. Afterwards, the sensitizer is eliminated from the lens, usually by natural processes or degradation. The elimination of the sensitizer returns the lens to a higher

stability state.

Other methods of altering the optical power of a lens use a lens that includes a polymer matrix and a chromophore that has multiple isomers that can be readily accessed via photoexcitation. In this method, the chromophore population is shifted from the ground state to a higher energy isomer by applying radiation at a first wavelength. This population is then exposed to a second wavelength of radiation which then acts to bleach the chromophore.

One example of such a compound is stilbene of Formula (III). As noted above, stilbene has cis- and trans-isomers in a first, natural, ratio under standard illumination. Stilbene photodegrades from the cis isomer, which is not the ground state. Rather, the trans isomer is the ground state for stilbene. By applying radiation at a first wavelength, the population of stilbene can be shifted to include more of the cis isomer relative to the amount of the trans isomer. Put another way, the ratio of cis-stilbene to trans-stilbene is increased to a second ratio by radiation at a first wavelength. Then, this population with the increased second ratio is exposed to radiation at a second wavelength. This causes photobleaching to occur in the exposed portion of the lens and changes the refractive index of the exposed portion of the lens, thereby altering the optical power of the lens. The first wavelength radiation generally has lower energy than the second wavelength radiation. As a result of this dual exposure, a higher degree of bleaching can be achieved than would be expected based solely on the application of the higher energy radiation.

In this regard, it is contemplated that a first portion of the lens is exposed to radiation at the first wavelength, and a second portion of the lens is exposed to radiation at the second wavelength. The second portion completely overlaps the first portion, or in other words the first portion can be of a larger area than the second portion.

The first ratio of higher energy isomer to ground state isomer may be from about $1 \times 10^{-6}$:1 to about 1:1. The resulting second ratio of higher energy isomer to ground state isomer may be from about 0.1:1 to about 100:1.

The first wavelength may be from about 3000 nm to about 300 nm. The second wavelength may be from about 1000 nm to about 190 nm. In embodiments, the first wavelength is different from the second wavelength; usually the second wavelength is shorter (i.e. higher-energy).

Still other methods of altering the optical power of a lens require the use of a lens that includes a polymer matrix and photobleachable multiphoton chromophores. As described above with reference to **FIG. 9,** such chromophores must absorb more than one photon for bleaching to occur. Incident light intensity is too low to cause bleaching. However, under high-intensity exposure to radiation, bleaching will occur. Thus, when a portion of the lens is exposed to radiation, photobleaching will occur in the exposed portion of the lens and change the refractive index of the exposed portion of the lens, thereby altering the optical power of the lens.

Here we distinguish between a two-photon process (as illustrated in **FIG. 9B**) and a multiphoton process (as illustrated in **FIG. 9C**). The process in **FIG. 9B** utilizes two photons of the same wavelength and so this process can occur under illumination by a single laser source. The process of **FIG. 9C** utilizes two photons of the same or different wavelengths to excite the molecule. Two-photon absorption is thus a special case of multiphoton absorption. In some literature the process of **FIG. 9B** is referred to as degenerate two-photon absorption and the process of **FIG. 9C** when the photons have different wavelengths is referred to as a non-degenerate two-photon processes.

One distinguishing difference between the two processes is that all chromophores are capable of multiphoton absorption, while all chromophores may not possess a measurable two photon absorption strength. Two-photon absorption is described in Advanced Materials p.4641 (2008), and J. Phys. Chem. A, p.105 (2011). These articles suggest that many of the potential chromophores listed above may have strong two-photon absorption. Some additional two-photon chromophores are illustrated below as Figures (A)-(E):

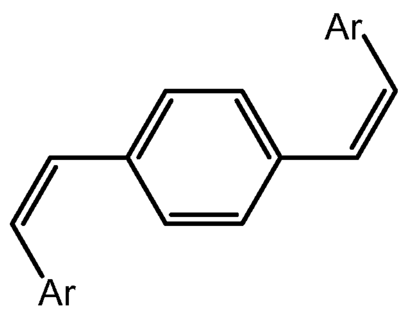

Figure (A)

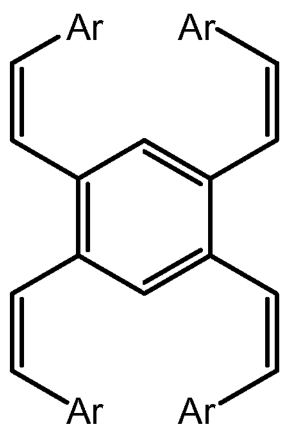

Figure (B)

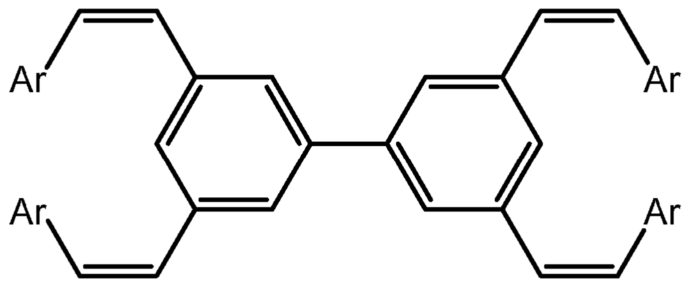

Figure (C)

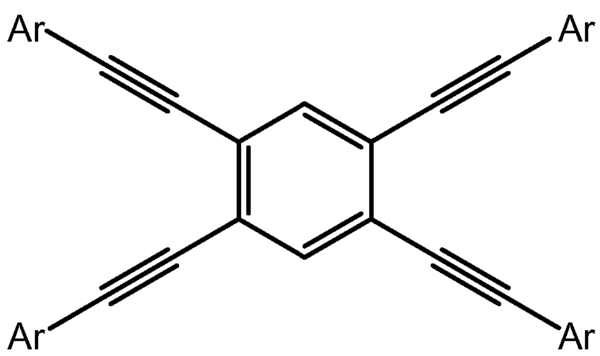

Figure (D)

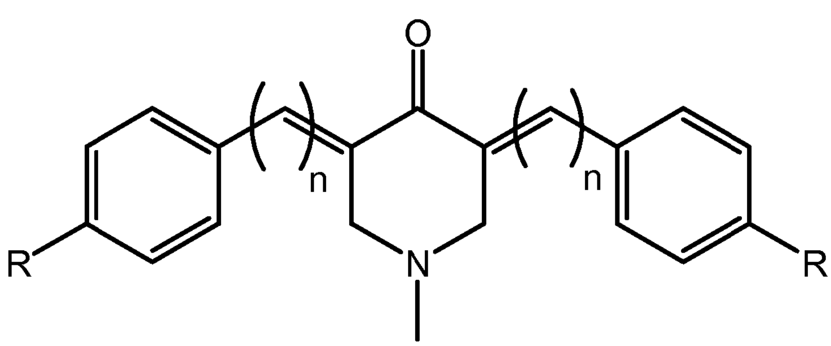

Figure (E)

wherein each Ar is an aryl or substituted aryl; each R is alkyl; and n reflects the number of double bonds in the conjugated

bridge and can be from 1 to 4.

In some embodiments, two or more multiphoton chromophores may be included in the lens.

In some embodiments, high intensity laser pulses may be utilized for the exposure to radiation. The control of the pulse shape can be used to control the distribution of isomers and of the photodegradation product.

While the methods described above are useful in adjusting the optical power of the lens, it should be noted that it is also desired that such lens are stable afterwards under normal illumination for a prolonged period of time. Thus, the lens and/or the chromophores can be modified in at least two ways. First, referring again to **FIG. 10,** the lens **200** may be considered as having an inner surface **214** and an outer surface **212.** An ultraviolet radiation absorbing layer **220** is present on the outer surface, and no such layer is present on the inner surface (i.e. layer **230** is not present). The lens is exposed to the radiation that causes photobleach on the inner surface **214.** Thus, the lens can be modified in a controlled manner and the UV absorbing layer **220** reduces or prevents such modification from occurring in standard illumination. Second, the chromophore can be selected to also meet the additional requirement that the bleached chromophore (i.e. in its bleached state) has a stronger absorption at the bleaching wavelength than the photobleachable chromophore (i.e. prior to being bleached). In this way, the bleached chromophore acts as a UV absorber and works to limit the amount of additional bleaching that can accur from natural illumination These two modifications should increase the stability of the lens.

Use of the various methods described above are specifically contemplated for use with intraocular lenses and with contact lenses. Contact lenses are generally made from biocompatible polymers which do not damage the ocular tissue and ocular fluid during the time of contact. In this regard, it is known that the contact lens must allow oxygen to reach the cornea. Extended periods of oxygen deprivation causes the undesirable growth of blood vessels in the cornea. "Soft" contact lenses conform closely to the shape of the eye, so oxygen cannot easily circumvent the lens. Thus, soft contact lenses must allow oxygen to diffuse through the lens to reach the cornea.

Another ophthalmic compatibility requirement for soft contact lenses is that the lens must not strongly adhere to the eye. The consumer must be able to easily remove the lens from the eye for disinfecting, cleaning, or disposal. However, the lens must also be able to move on the eye in order to encourage tear flow between the lens and the eye. Tear flow between the lens and eye allows for debris, such as foreign particulates or dead epithelial cells, to be swept from beneath the lens and, ultimately, out of the tear fluid. Thus, a contact lens must not adhere to the eye so strongly that adequate movement of the lens on the eye is inhibited.

Suitable materials for contact lenses are well known in the art. For example, polymers and copolymers based on 2-hydroxyethyl methacrylate (HEMA) are known, as are siloxane-containing polymers that have high oxygen permeability, as well as silicone hydrogels. Any suitable material can be used for the polymer matrix of a contact lens to which the methods described herein can be applied.

Aspects of the present disclosure may be further understood by referring to the following examples. The examples are merely for further describing various aspects of the devices and methods of the present disclosure and are not intended to be limiting embodiments thereof. None of the following examples are according to the invention. They are for reference only.

## EXAMPLES

Experimental measurements were performed to verify that the refractive index changed upon crosslinking and without the presence of free monomer. The experiments also showed that the refractive index change was controllable, reproducible, and adjustable.

A series of experiments were also performed to verify the amount of change in the refractive index possible with photobleaching, and also that photobleaching could occur in an aqueous environment.

## EXAMPLE 1

A solution of 37.5% SARTOMER CN990, 59.4% SARTOMER SR344 and 3.1% DAROCUR 4265 was created (composition was based on weight fraction of the components). SARTOMER CN990 is a siliconized urethane acrylate oligomer. SARTOMER SR344 is a polyethylene glycol diacrylate having a molecular weight of 508. DAROCUR 4265 is a photoinitiator mixture of 50 weight percent diphenyl (2,4,6-trimethylbenzoyl) phosphine oxide and 50 weight percent 2-hydroxy-2-methyl-1-phenyl-1-propanone. DAROCUR 4265 has absorption peaks at 240, 272, and 380 nm in methanol.

The refractive index of the solution was measured to be 1.4717. The solution was then cast onto a slide and exposed to 10 seconds of UV light from a lamp source. The refractive index of the film was then measured using a Metricon Model 2010 Prism Coupler, and was determined to be 1.4747. Additional UV exposure of 5 seconds gave a refractive index of n=1.4852. Further UV exposure of 15 seconds gave a refractive index of n=1.4868. In other words, as the amount of UV exposure increased, the amount of crosslinking increased and thus the refractive index increased.

**EXAMPLE 2**

The solution of Example 1 was again created, except the DAROCUR 4265 was replaced with IRGACURE 2959, which is sensitive to 254 nm UV. The solution was cast onto a plate and exposed to UV light. After 2 seconds exposure, the refractive index was measured with the prism coupler and found to be n=1.4668, with the film not being fully cured in the center. An additional 5 second UV exposure led to the refractive index being measured as n=1.485.

**EXAMPLE 3**

A third experiment was performed to better evaluate the exposure-dependence of the refractive index. A solution similar to that of Example 1 was prepared and cast onto a slide. The film then underwent several cycles of UV exposure and refractive index measurement. The results are shown in **FIG. 12.** Below 8 seconds exposure, the film was not fully set. The data is also summarized in Table 1.

Table 1.

| Time (sec) | Refractive Index |
|---|---|
| 8 | 1.4799 |
| 10 | 1.4804 |
| 15 | 1.4835 |
| 20 | 1.4887 |
| 25 | 1.489 |

The results indicated that the refractive index could be changed by a range of about 0.01 after exposure of about 25 seconds. This value of index change was selected as providing approximately a 10% change in lens power (as shown in **FIG. 2**).

**EXAMPLE 4**

A solution of VC60 in polymethylmethacrylate (PMMA) was cast as a film and dried at 80°C for 10 minutes. The refractive index of the film was 1.4909. The film was then exposed to 254 nm radiation for 1 minute. The refractive index was then measured to be 1.5016. After further exposure (30 minutes total) the refractive index was 1.5036. Absorbance measurements showed -50% decrease in absorbance due to the chromophore.

**EXAMPLE 5**

A solution of EC24 in PMMA was cast as a film and dried at 80°C for 10 minutes. The refractive index of the film was measured as n=1.5014. The film was then exposed to 254 nm radiation for 30 minutes. The refractive index was then measured as n=1.4924.

**EXAMPLE 6**

EC24 was then diffused into an ACUVUE lens (Johnson & Johnson Vision Care, Inc.). The lens was partially masked, then exposed to 254 nm UV light for 30 minutes. The chromophore bleached, but over time the demarcation line between the masked and unmasked portions of the lens blurred. This may be attributable to migration of the chromophore in the lens.

The experiment was then repeated by diffusing EC24 into two separate ACUVUE lenses. The first lens was kept as a control, and exhibited very uniform red color, consistent with an absorption maximum near 510 nm for EC24 in the lens. The second lens was exposed to the UV light for 30 minutes. At the end of this exposure, the second lens exhibited no color and was completely transparent.

**EXAMPLE 7**

The transmission spectra of an ACUVUE lens was measured. EC24 was then diffused into the lens, and the transmission spectrum was measured again. Finally, the lens was bleached and the transmission spectrum was measured

a third time. The results are shown in **FIG. 13** and Table 2.

Table 2.

| Wavelength (nm) | %T hydrated lens | %T EC24 doped lens | %T bleached lens |
|---|---|---|---|
| 350 | 10.3 | 4.5 | 21 |
| 360 | 10.7 | 11.7 | 19.4 |
| 370 | 28.4 | 30.1 | 36.4 |
| 380 | 63.9 | 52.6 | 60.9 |
| 390 | 82.5 | 62 | 72.1 |
| 400 | 87 | 64 | 72.8 |
| 410 | 87.9 | 64.4 | 75.9 |
| 420 | 88.3 | 64.5 | 76.4 |
| 430 | 88.4 | 64.5 | 76.7 |
| 440 | 88.8 | 64.2 | 77.2 |
| 450 | 88.8 | 64.1 | 77.6 |
| 460 | 89 | 63.5 | 77.6 |
| 470 | 89 | 62.6 | 77.8 |
| 480 | 89.1 | 61.9 | 78.7 |
| 490 | 89.2 | 61.3 | 80 |
| 500 | 89.1 | 60.6 | 81.2 |
| 510 | 88.9 | 60.3 | 81.6 |
| 520 | 88.8 | 60.6 | 82.2 |
| 530 | 88.8 | 61.1 | 82.7 |
| 540 | 88.9 | 62.4 | 83.5 |
| 550 | 88.9 | 64.1 | 83.9 |
| 560 | 88.9 | 66.2 | 84.5 |
| 570 | 88.9 | 67.8 | 84.9 |
| 580 | 88.9 | 69.3 | 85.5 |
| 590 | 88.8 | 70 | 86 |
| 600 | 89 | 70.6 | 86.6 |

Note that the EC24 doped lens shows a transmission minimum close to 510 nm, while the absorption maximum of EC24 in dioxane was measured to be 503 nm. This indicates that the EC24 is present in the doped lens. The photobleached lens has weaker absorption and no longer has the absorption at 510 nm, indicating that the photobleaching process has altered the chemistry of EC24.

The devices and methods of the present disclosure have been described with reference to exemplary embodiments. It is intended that the exemplary embodiments be construed as including other modifications and alterations that may come within the scope of the appended claims.

## Claims

**1.** A method of altering the optical power of a lens, comprising:

providing a lens having a first optical power, the lens comprising a polymer matrix having photobleachable

chromophores;
exposing a portion of the lens to radiation, causing photobleaching to occur in the exposed portion of the lens and changing the refractive index of the exposed portion of the lens, thereby altering the optical power of the lens to a second different optical power,
**characterized in that** a radical scavenger is dispersed in the polymer matrix.

**2.** The method of claim 1, wherein the lens is a contact lens.

**3.** The method of claim 1, wherein the lens has an inner surface and an outer surface, the outer surface includes an ultraviolet radiation absorbing layer, and the exposing of the lens occurs from the inner surface.

**4.** The method of claim 1, wherein the photobleachable chromophores contain a malonitrile moiety or are stilbene chromophores, or are azobenzene chromophores.

**5.** The method of claim 1, wherein the radical scavenger is selected from the group consisting of hindered phenols, secondary aromatic amines, hydroquinones, benzoquinones, piperazines, polybutadienes, and mercaptans.

**6.** The method of claim 1, wherein the radical scavenger is present in an amount of about 0.01 to about 10 wt% by weight of the lens.

## Patentansprüche

**1.** Verfahren zum Ändern der optischen Brechkraft einer Linse, umfassend:

Bereitstellen einer Linse mit einer ersten optischen Brechkraft, wobei die Linse eine Polymermatrix mit photobleichbaren Chromophoren umfasst;
Aussetzen eines Teils der Linse einer Strahlung, wodurch das Auftreten von Photobleichung in dem bestrahlten Teil der Linse bewirkt wird und der Brechungsindex des bestrahlten Teils der Linse geändert wird, wodurch die optische Brechkraft der Linse auf eine zweite unterschiedliche optische Brechkraft geändert wird,
**dadurch gekennzeichnet, dass** ein Radikalfänger in der Polymermatrix dispergiert ist.

**2.** Verfahren nach Anspruch 1, wobei die Linse eine Kontaktlinse ist.

**3.** Verfahren nach Anspruch 1, wobei die Linse eine innere Oberfläche und eine äußere Oberfläche aufweist, die äußere Oberfläche eine ultraviolette Strahlung absorbierende Schicht beinhaltet, und die Belichtung der Linse von der Innenfläche aus erfolgt.

**4.** Verfahren nach Anspruch 1, wobei die photobleichbaren Chromophore einen Malonitril-Anteil enthalten oder Stilben-Chromophore sind oder Azobenzol-Chromophore sind.

**5.** Verfahren nach Anspruch 1, wobei der Radikalfänger ausgewählt ist aus der Gruppe bestehend aus gehinderten Phenolen, sekundären aromatischen Aminen, Hydrochinonen, Benzochinonen, Piperazinen, Polybutadienen und Mercaptanen.

**6.** Verfahren nach Anspruch 1, wobei der Radikalfänger in einer Menge von etwa 0,01 bis etwa 10 Gew.-%, bezogen auf das Gewicht der Linse, vorliegt.

## Revendications

**1.** Procédé de modification du pouvoir optique d’une lentille, comprenant :

la fourniture d’une lentille ayant un premier pouvoir optique, la lentille comprenant une matrice polymère ayant des chromophores aptes au photoblanchiment ;
l’exposition d’une partie de la lentille à un rayonnement, provoquant un photoblanchiment dans la partie exposée de la lentille et changeant l’indice de réfraction de la partie exposée de la lentille, ce qui modifie le pouvoir optique de la lentille en un deuxième pouvoir optique différent,

**caractérisé en ce qu'**un capteur de radicaux est dispersé dans la matrice polymère.

2. Procédé selon la revendication 1, dans lequel la lentille est une lentille de contact.

3. Procédé selon la revendication 1, dans lequel la lentille a une surface interne et une surface externe, la surface externe inclut une couche absorbant le rayonnement ultraviolet, et l'exposition de la lentille a lieu à partir de la surface interne.

4. Procédé selon la revendication 1, dans lequel les chromophores aptes au photoblanchiment contiennent un groupement malonitrile ou sont des chromophores stilbène, ou sont des chromophores azobenzène.

5. Procédé selon la revendication 1, dans lequel le capteur de radicaux est sélectionné dans le groupe constitué de phénols encombrés, d'amines aromatiques secondaires, d'hydroquinones, de benzoquinones, de pipérazines, de polybutadiènes et de mercaptans.

6. Procédé selon la revendication 1, dans lequel le capteur de radicaux est présent en une quantité d'environ 0,01 à environ 10 % en poids de la lentille.

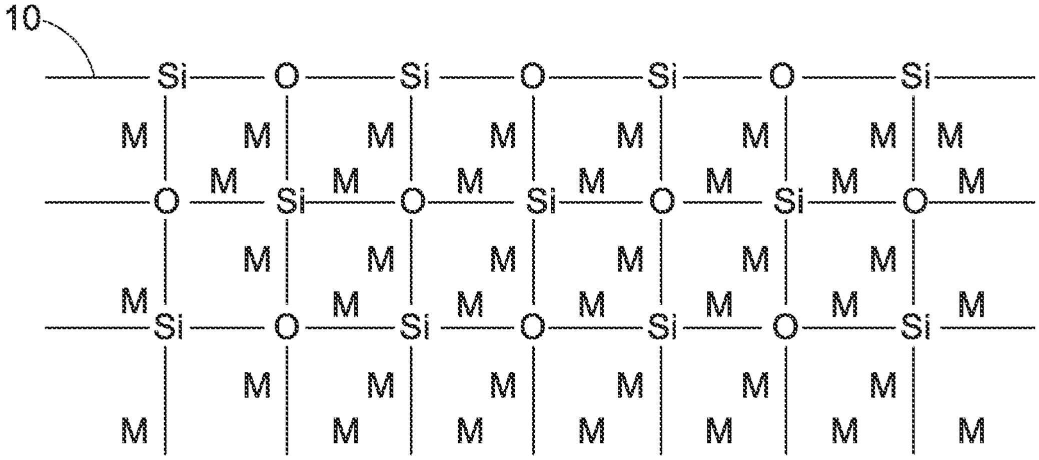


FIG. 1A

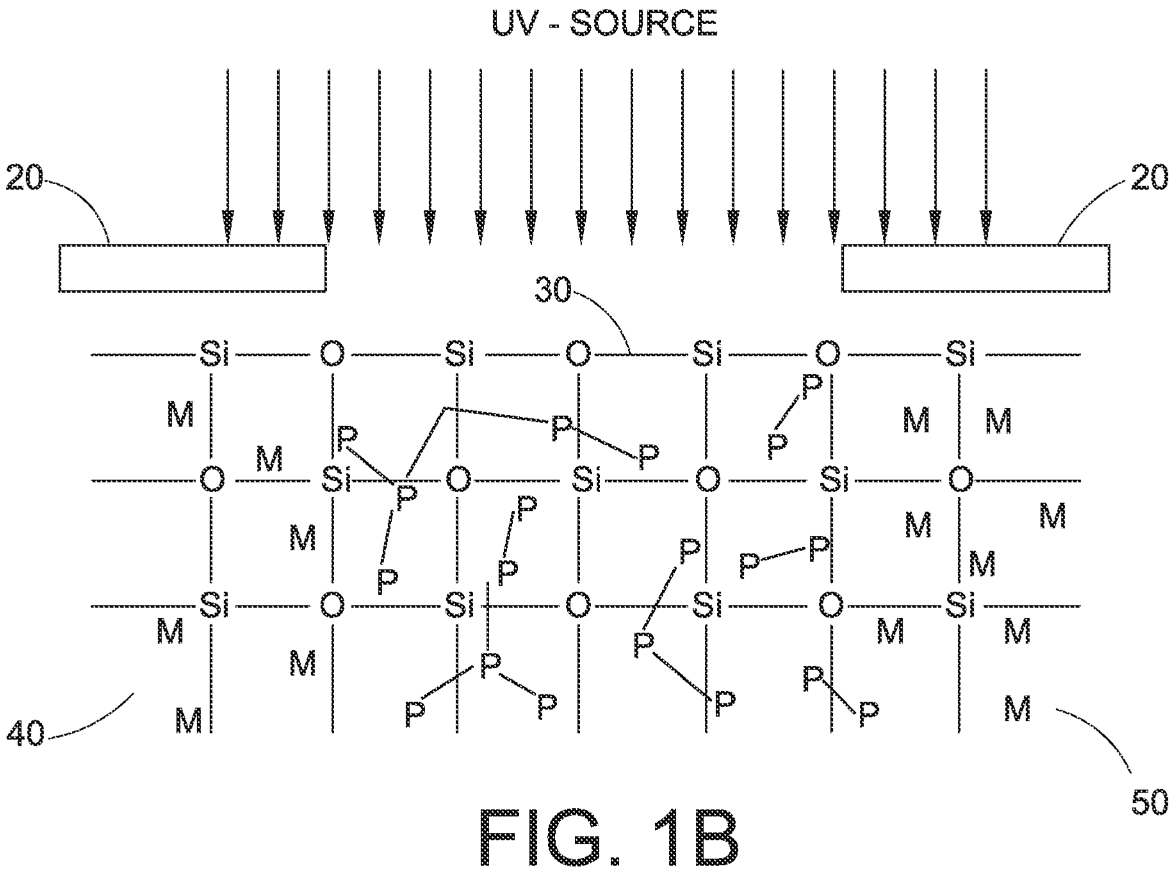


FIG. 1B

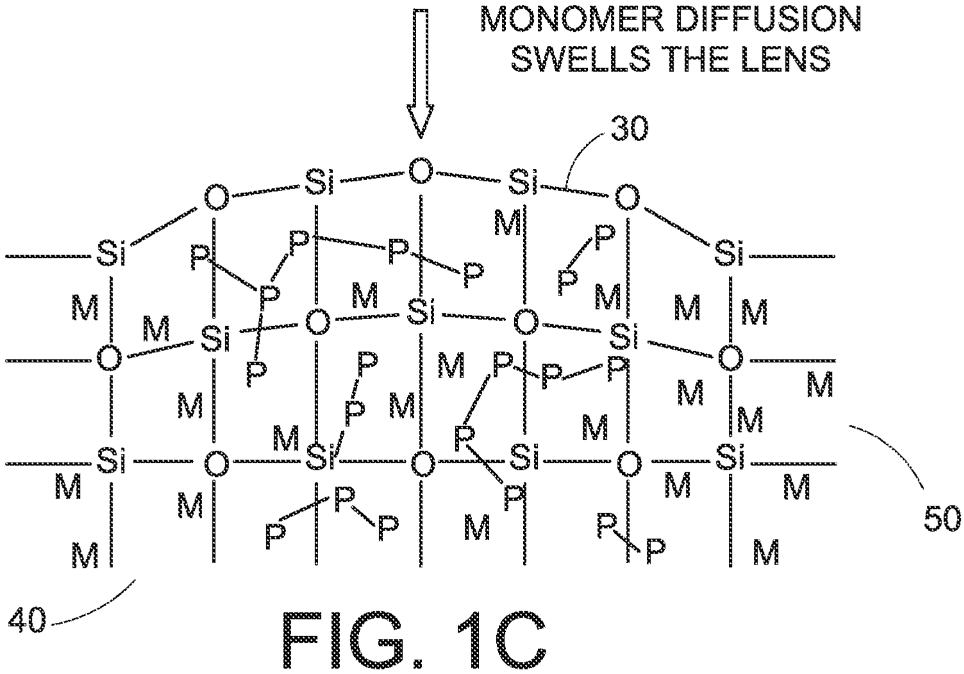


FIG. 1C

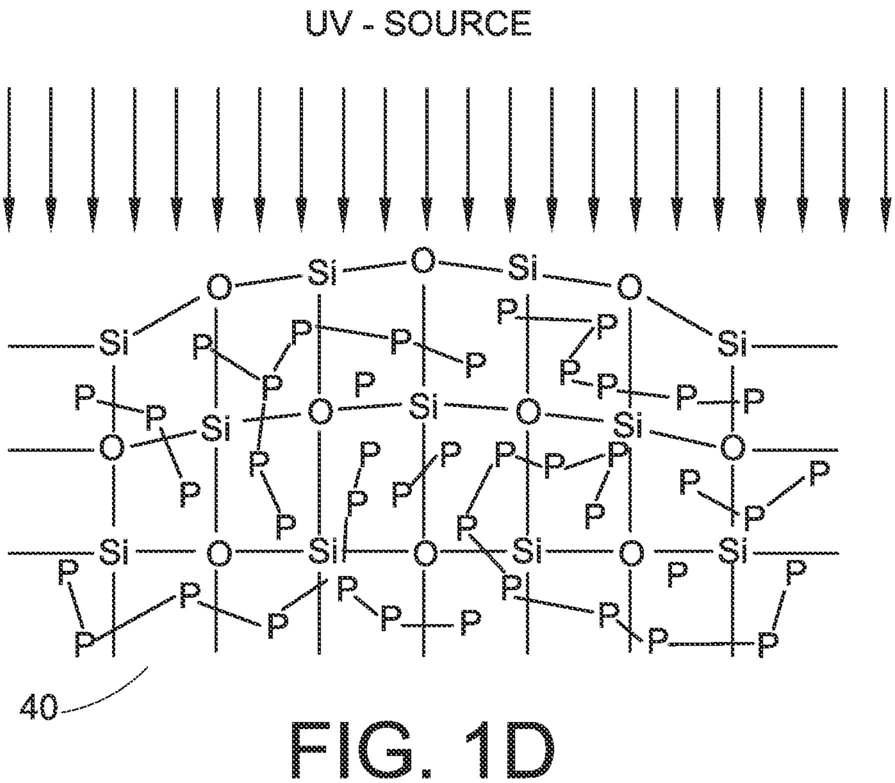


FIG. 1D

FIG. 2.

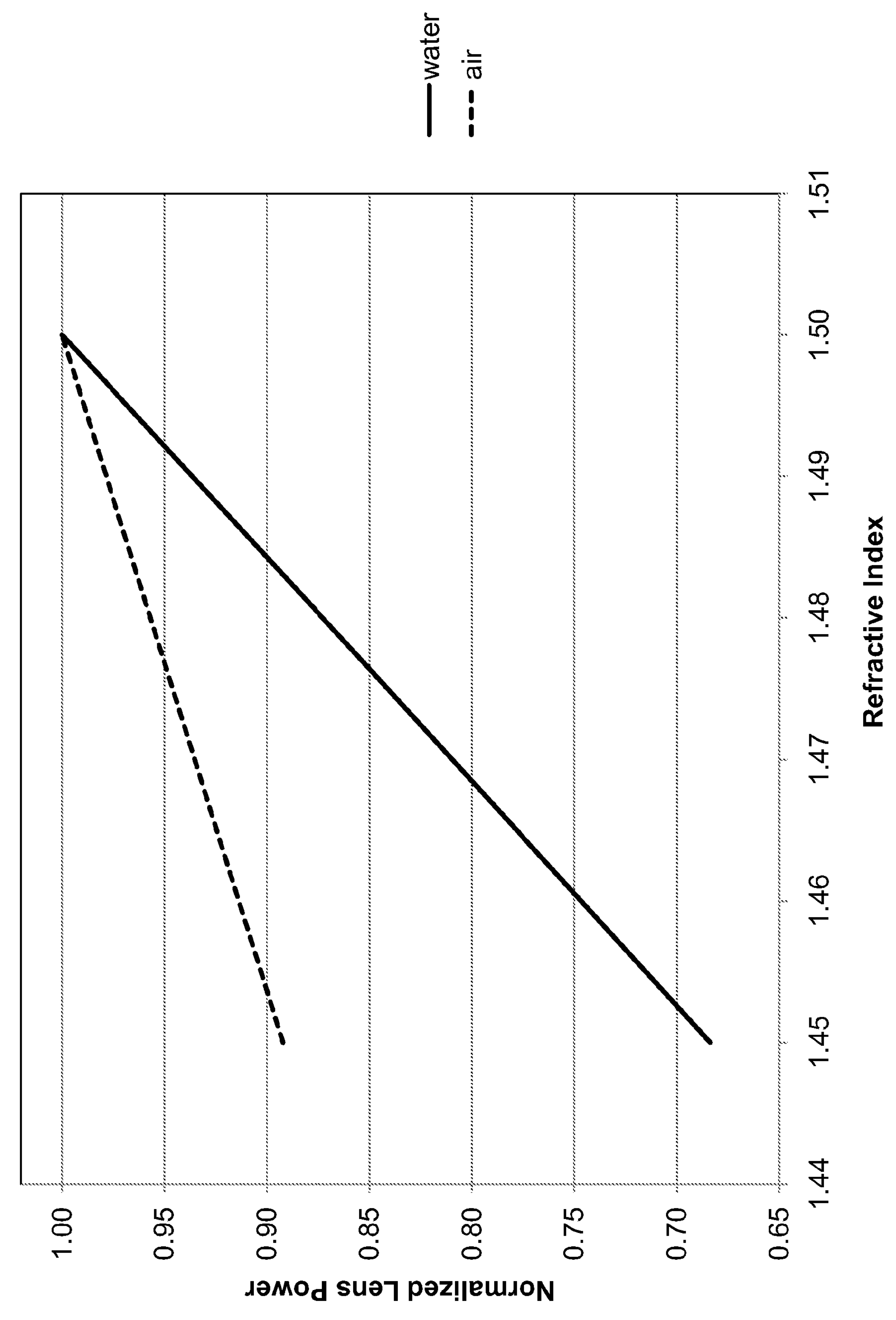
Normalized Lens Power
1.00
0.95
0.90
0.85
0.80
0.75
0.70
0.65
Refractive Index
1.44
1.45
1.46
1.47
1.48
1.49
1.50
1.51
water
air

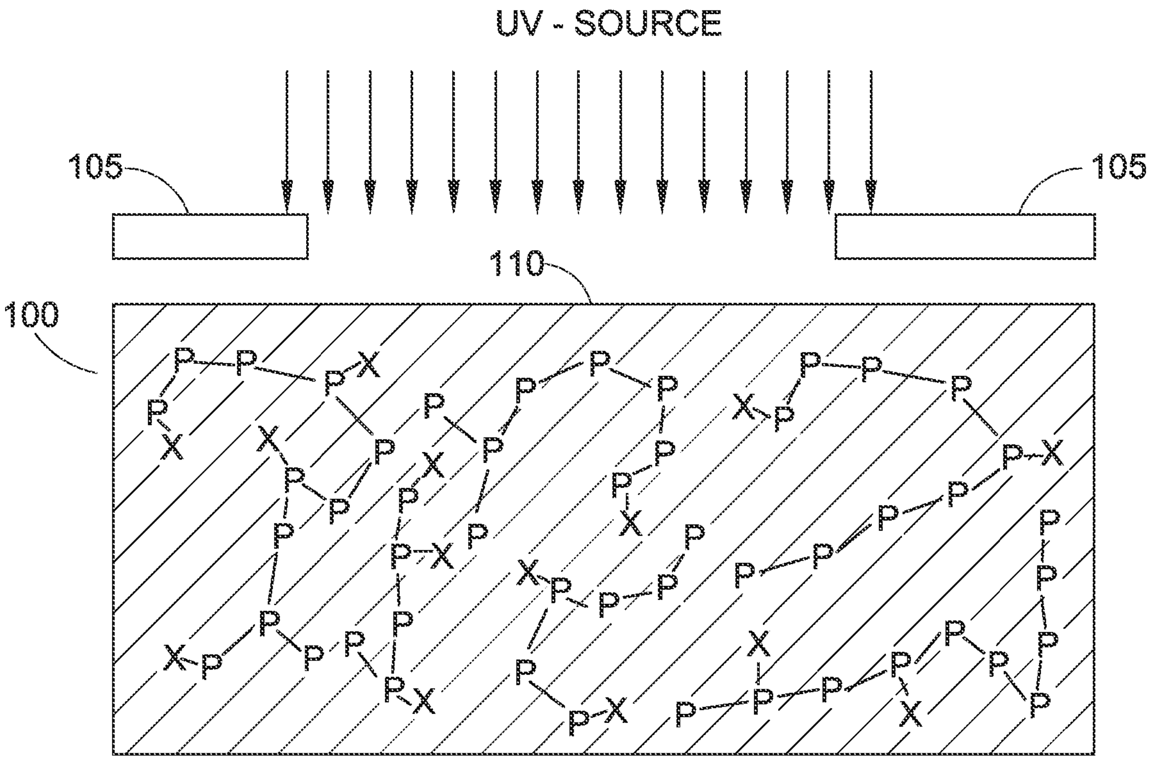


FIG. 3A

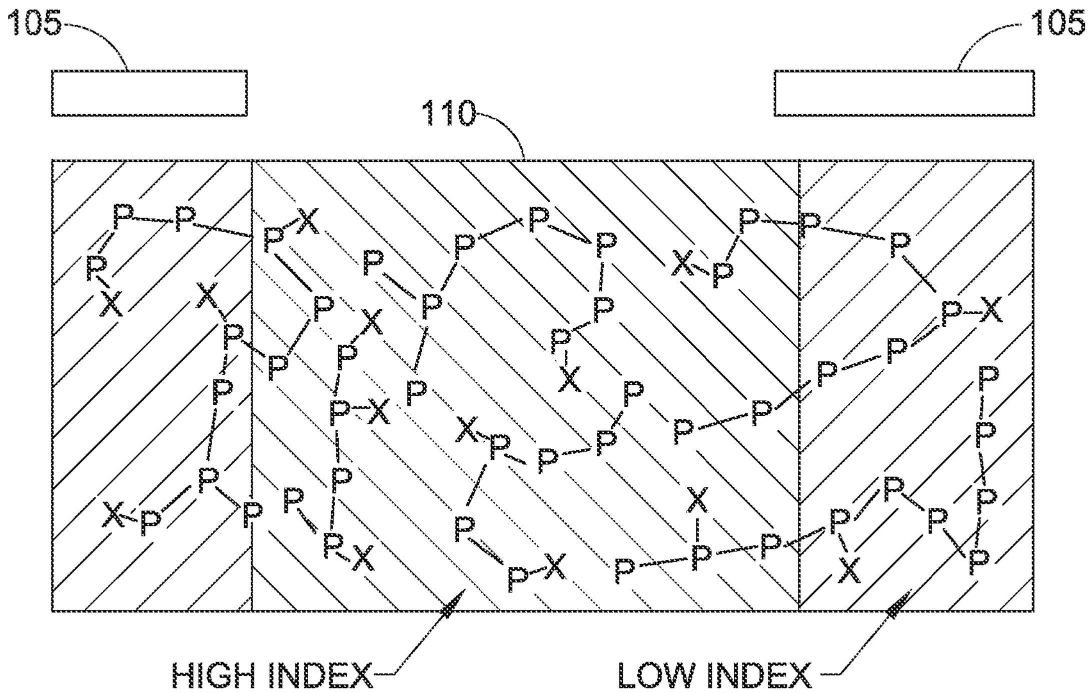


FIG. 3B

FIG. 4.

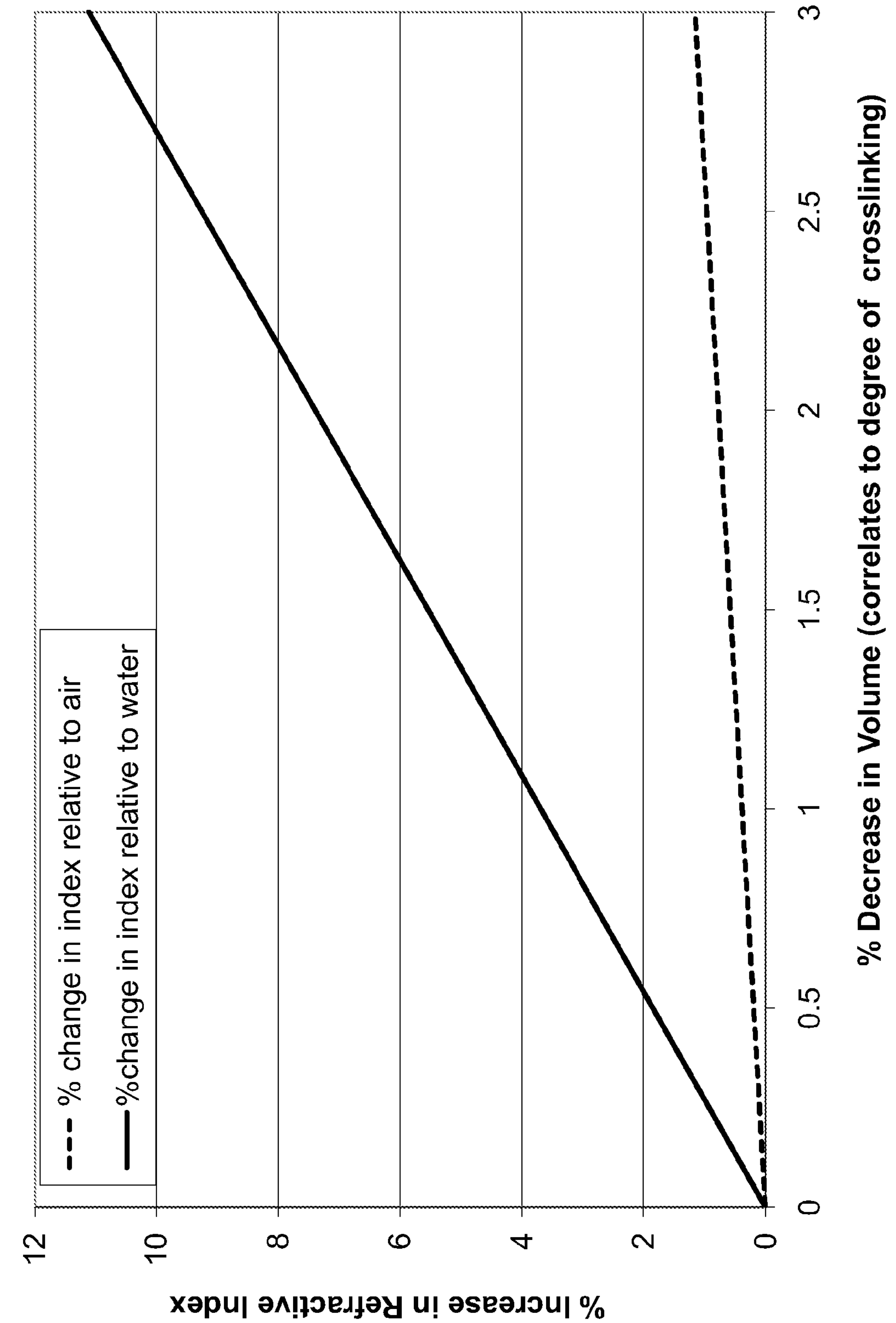
% change in index relative to air
%change in index relative to water
% Increase in Refractive Index
12
10
8
6
4
2
0
0
0.5
1
1.5
2
2.5
3
% Decrease in Volume (correlates to degree of crosslinking)

FIG. 5.

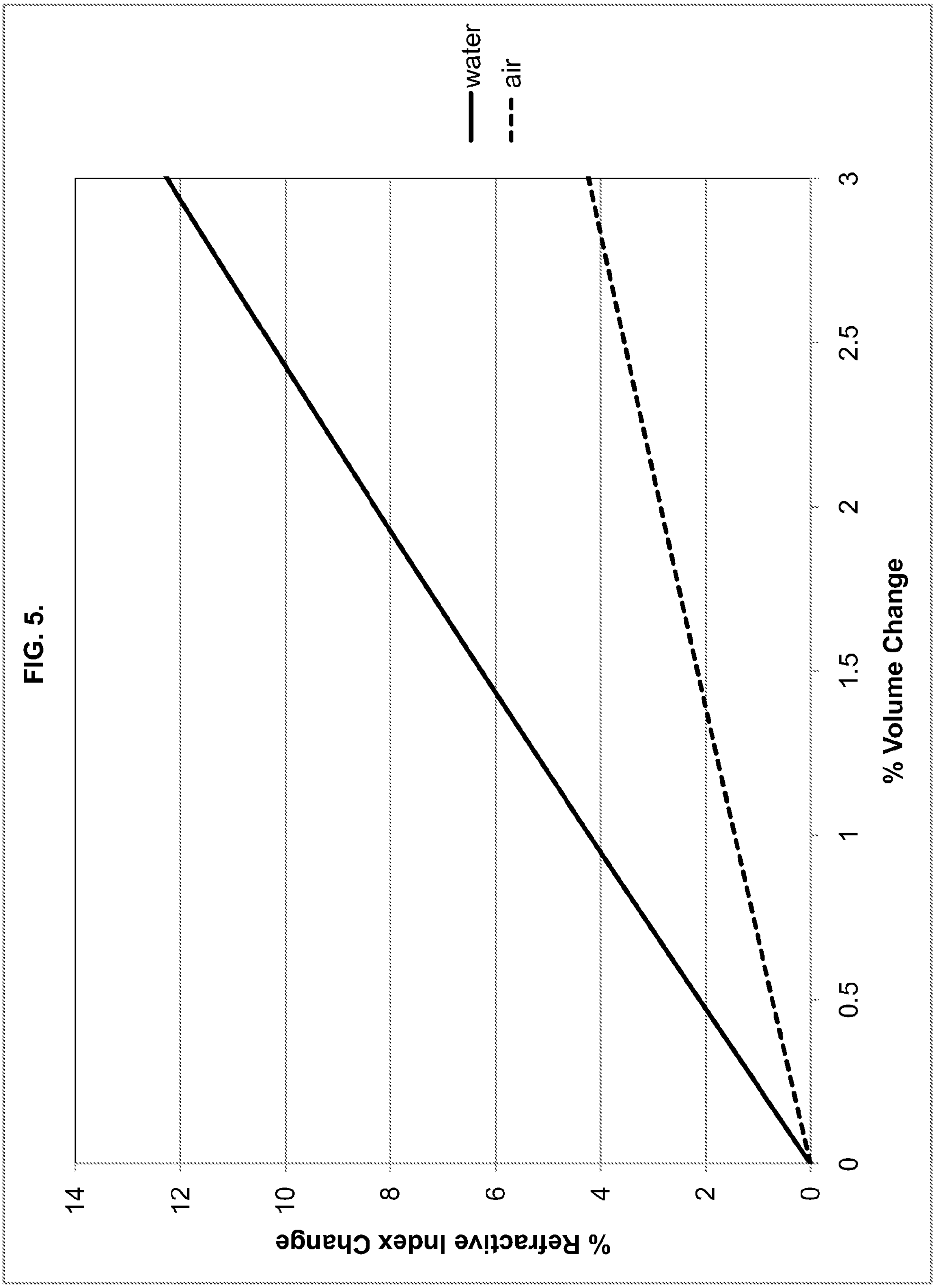
% Refractive Index Change
14
12
10
8
6
4
2
0
0
0.5
1
1.5
2
2.5
3
% Volume Change
water
air

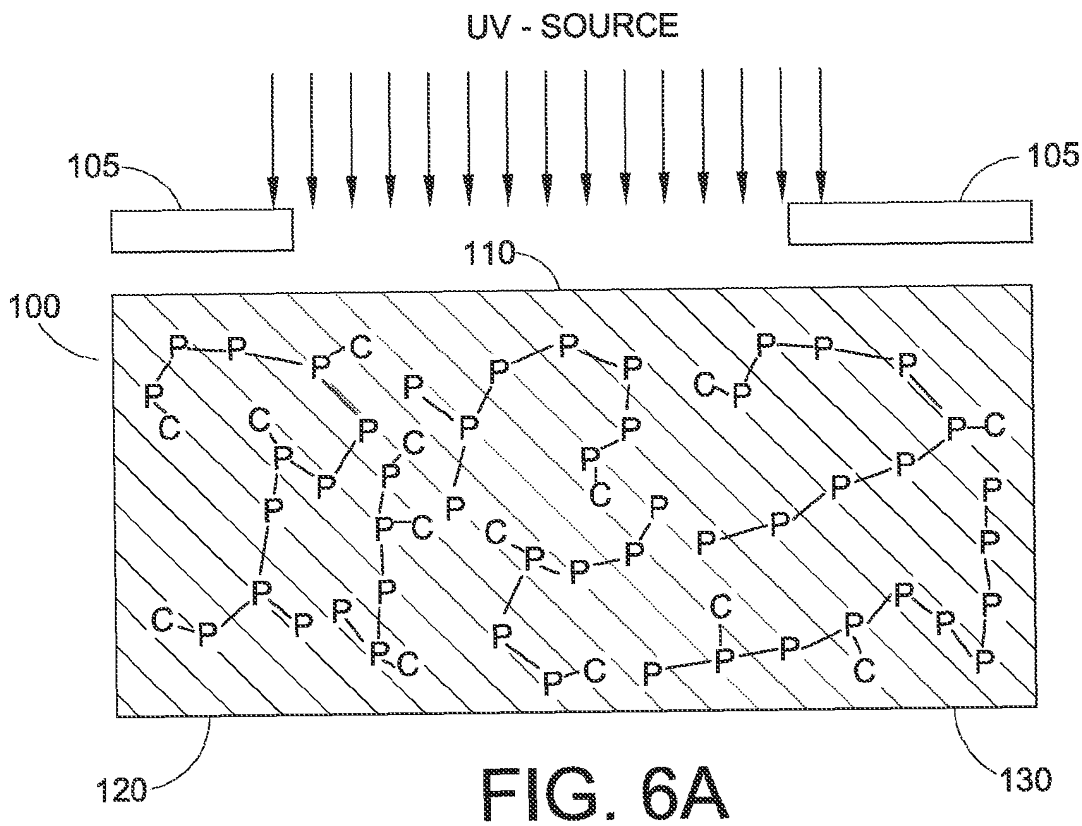
UV - SOURCE
105
105
110
100
120
130

FIG. 6A

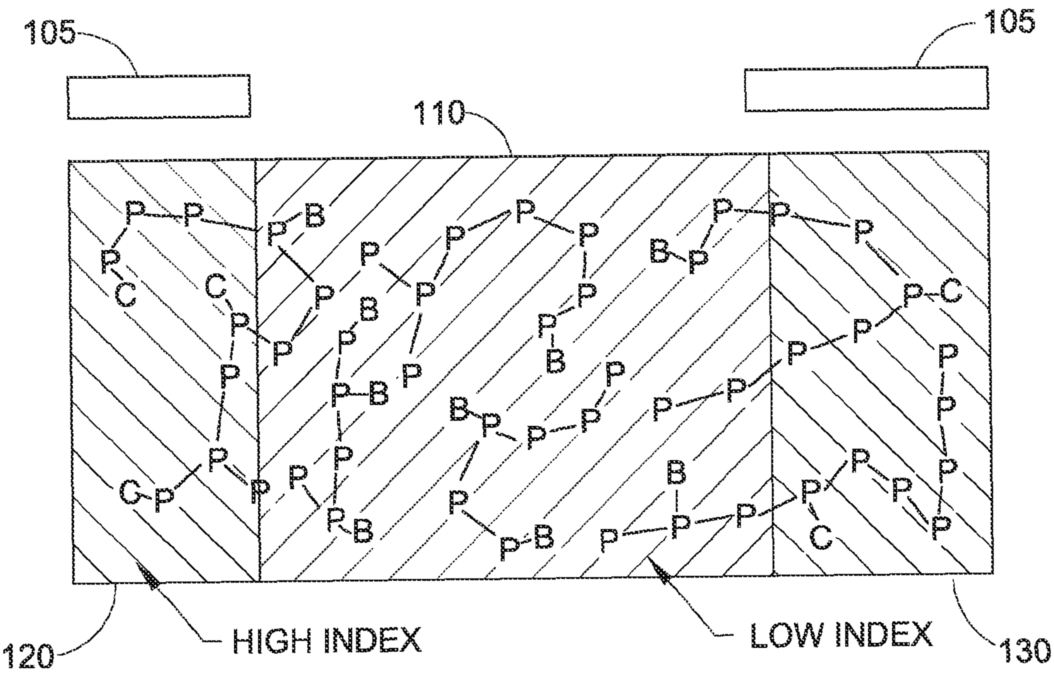
105
105
110
120
HIGH INDEX
LOW INDEX
130

FIG. 6B

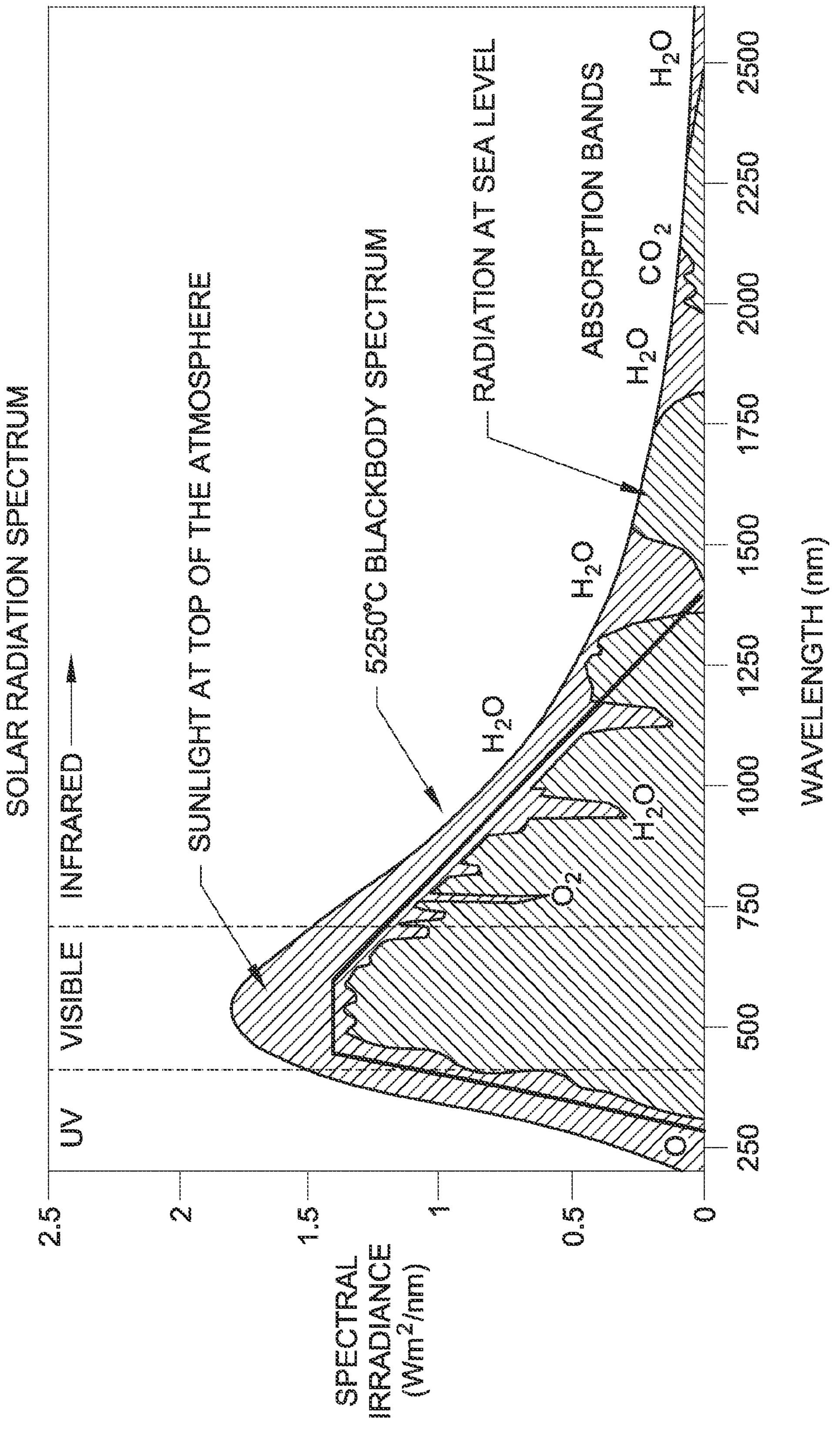
SOLAR RADIATION SPECTRUM
UV
VISIBLE
INFRARED
SUNLIGHT AT TOP OF THE ATMOSPHERE
5250°C BLACKBODY SPECTRUM
RADIATION AT SEA LEVEL
ABSORPTION BANDS
$H_2O$
$O_2$
$CO_2$
$O_3$
SPECTRAL IRRADIANCE ($Wm^2/nm$)
2.5
2
1.5
1
0.5
0
250
500
750
1000
1250
1500
1750
2000
2250
2500
WAVELENGTH (nm)

FIG. 7

FIG. 8.

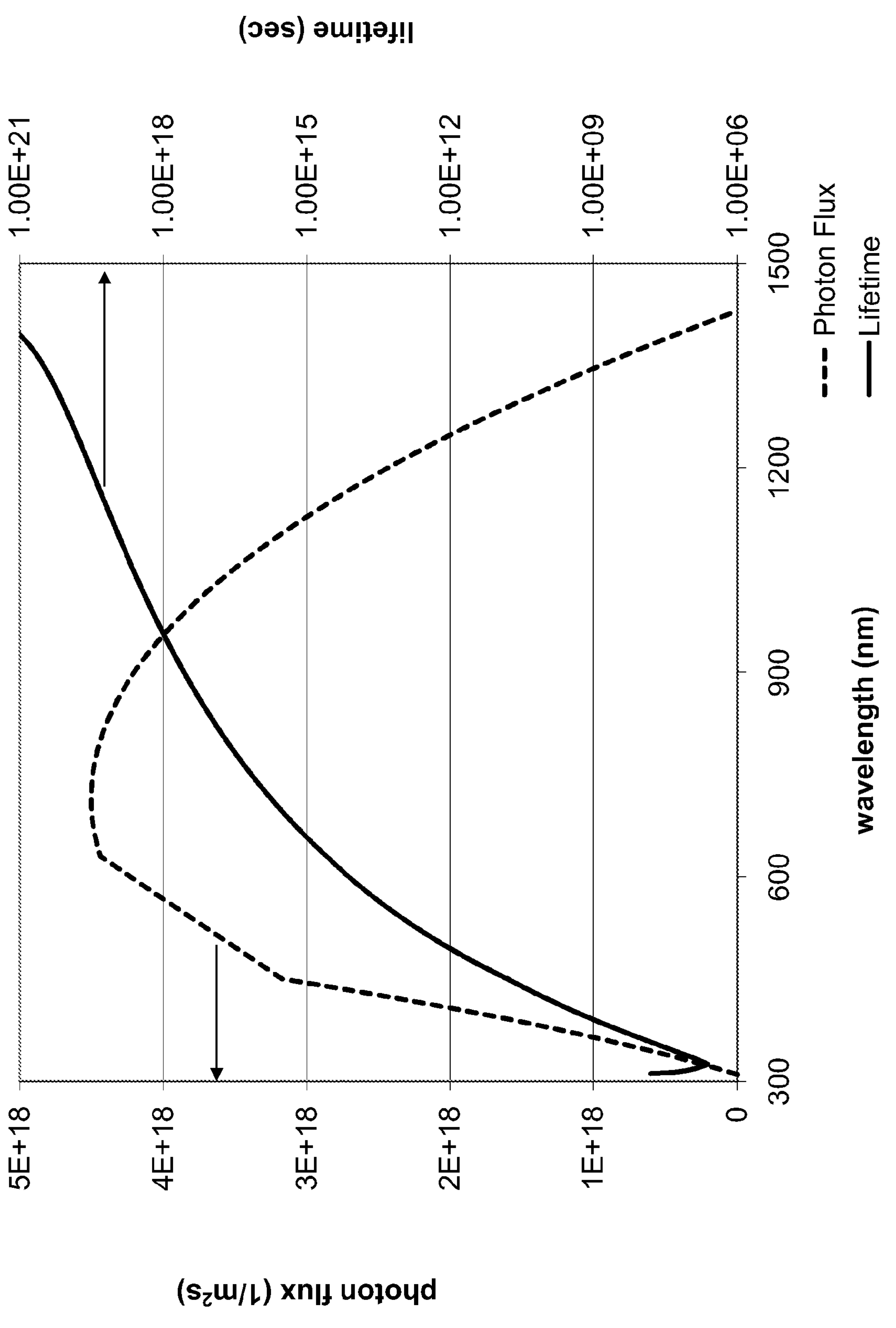
photon flux (1/m²s)
5E+18
4E+18
3E+18
2E+18
1E+18
0
lifetime (sec)
1.00E+21
1.00E+18
1.00E+15
1.00E+12
1.00E+09
1.00E+06
300
600
900
1200
1500
wavelength (nm)
Photon Flux
Lifetime

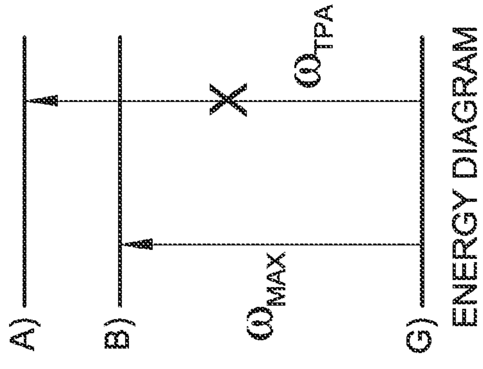


FIG. 9A

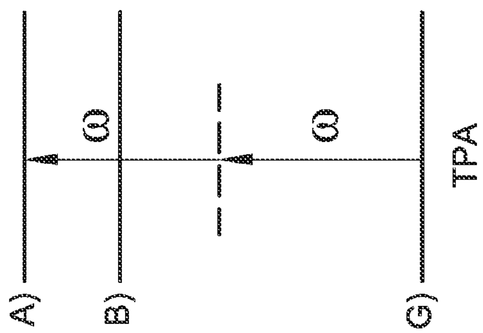


FIG. 9B

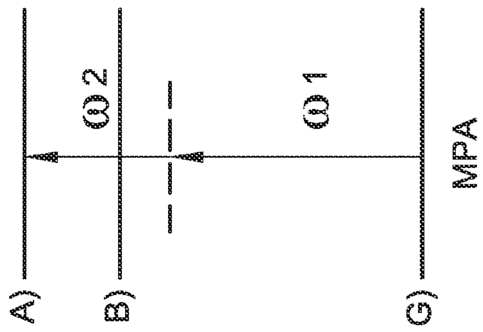


FIG. 9C

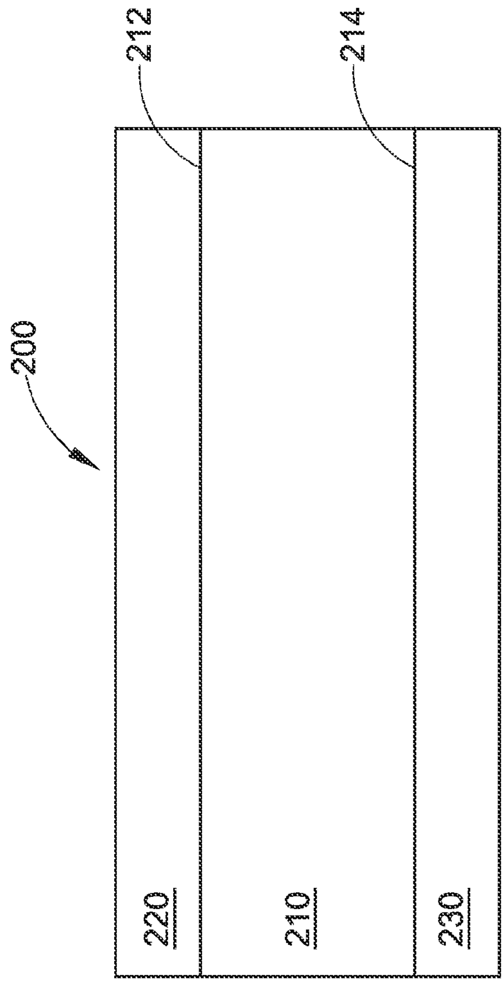


FIG. 10

FIG. 11.

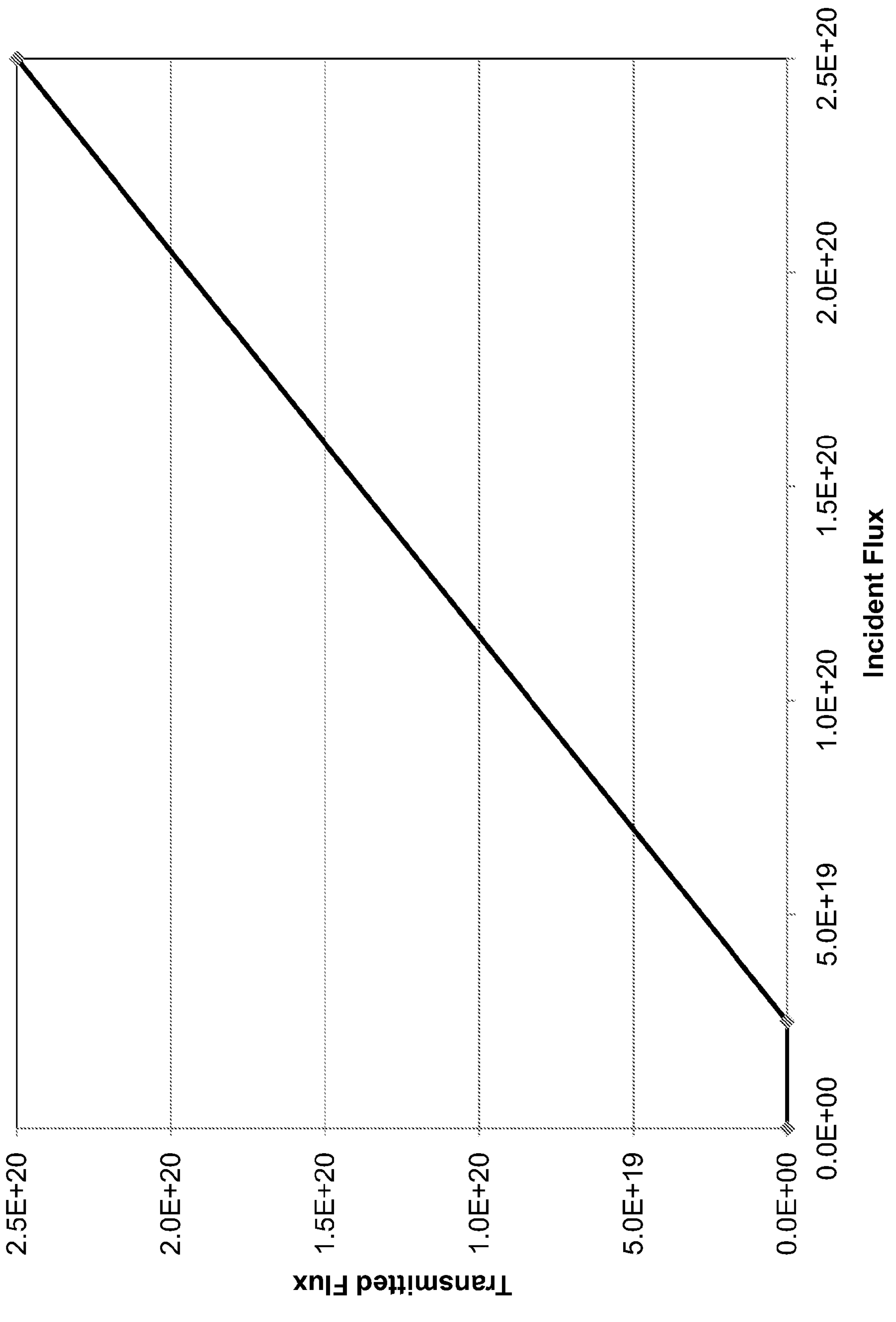
Transmitted Flux
2.5E+20
2.0E+20
1.5E+20
1.0E+20
5.0E+19
0.0E+00
0.0E+00
5.0E+19
1.0E+20
1.5E+20
2.0E+20
2.5E+20
Incident Flux

FIG. 12.

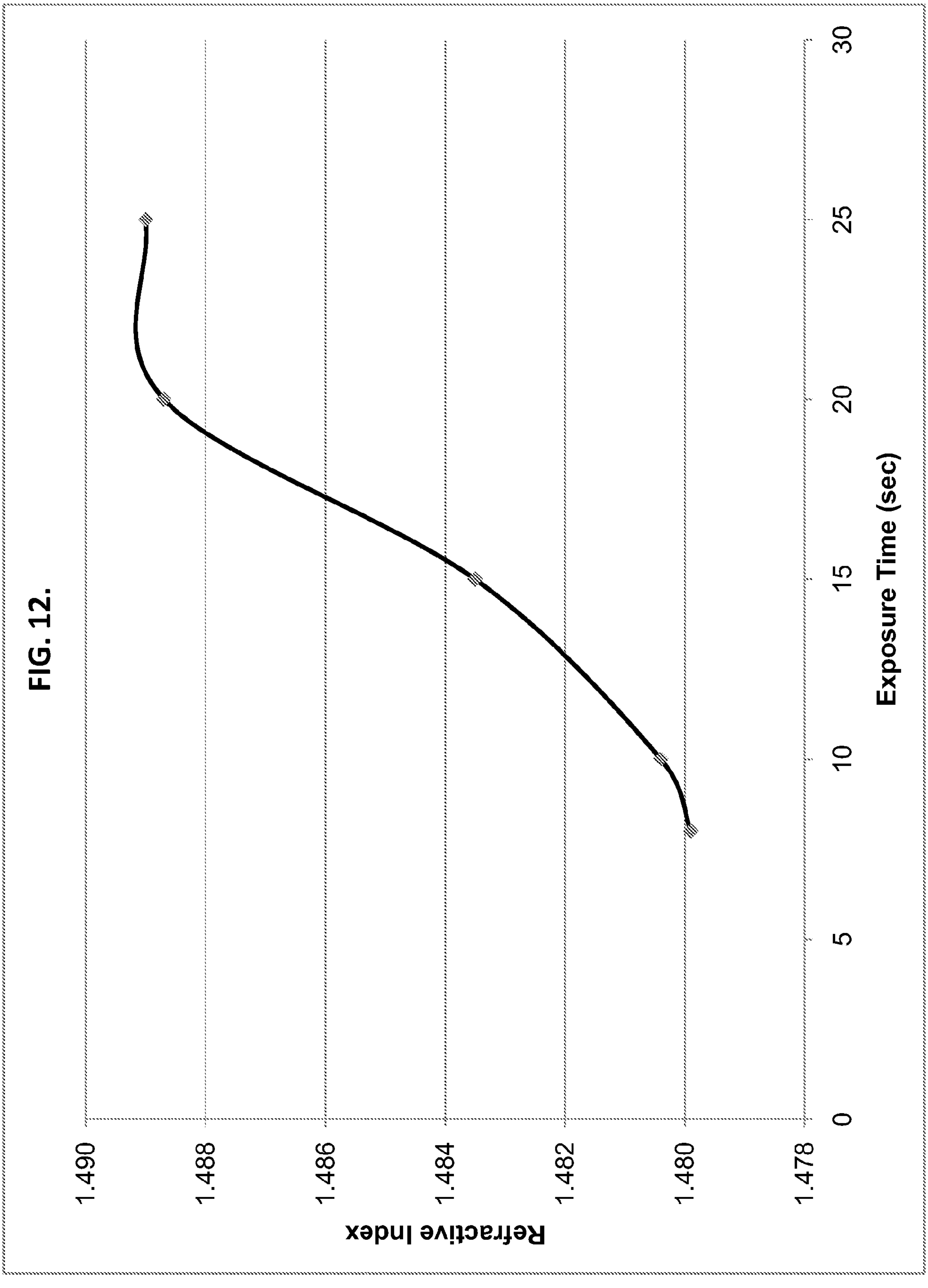
Refractive Index
1.490
1.488
1.486
1.484
1.482
1.480
1.478
0
5
10
15
20
25
30
Exposure Time (sec)

FIG. 13.

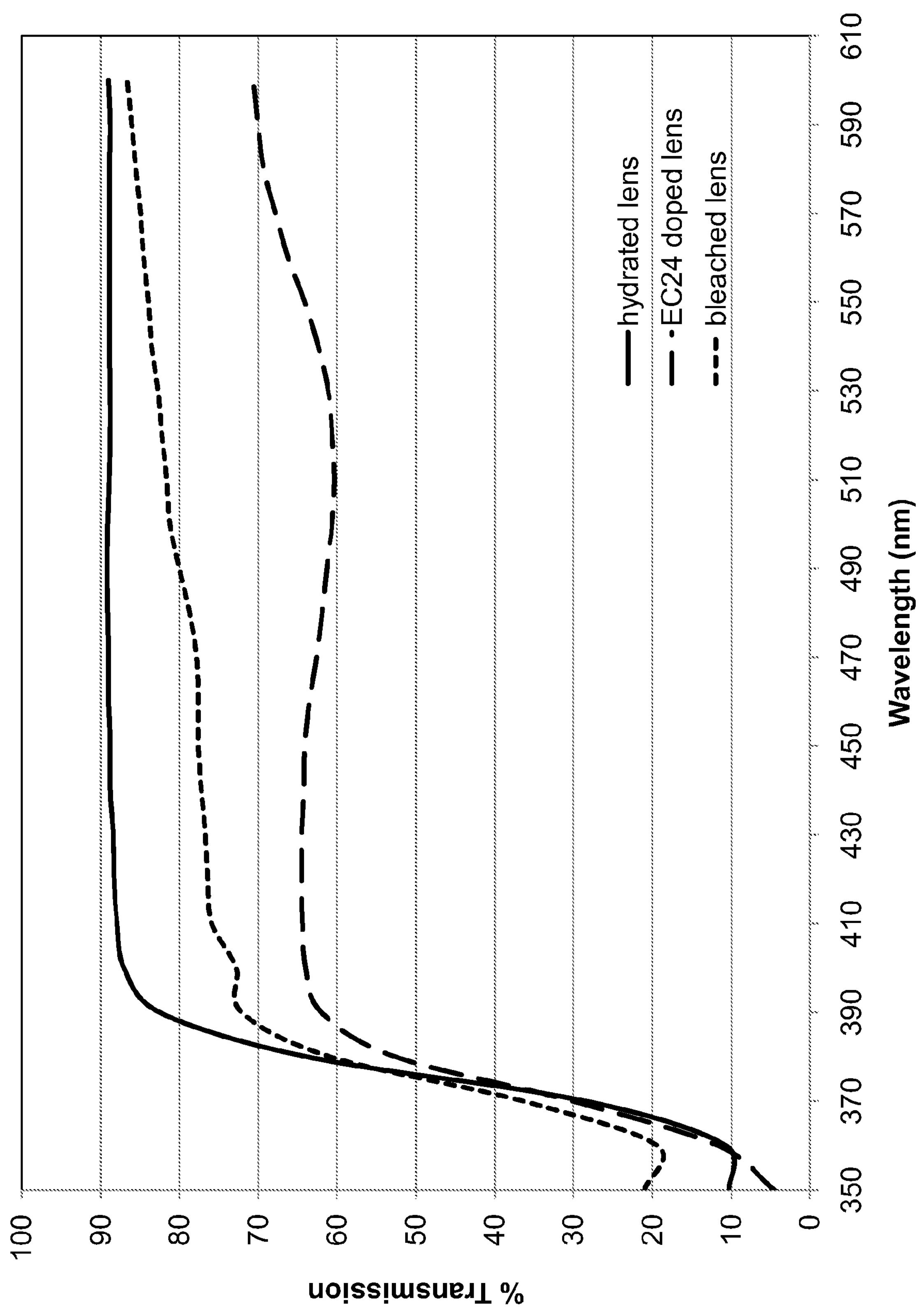
% Transmission
100
90
80
70
60
50
40
30
20
10
0
350
370
390
410
430
450
470
490
510
530
550
570
590
610
Wavelength (nm)
hydrated lens
EC24 doped lens
bleached lens

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61876845
- US 7134755 B
- US 20110144747 A1
- US 6244707 B
- US 4719248 A


### Non-patent literature cited in the description


- **DEXTER.** UV Stabilizers. *Kirk-Othmer Encyclopedia of Chemical Technology,* 1983, vol. 23, 615-627
- **MILLAN.** *Polymer,* 2005, vol. 46, 5556-5568
- *Trans. Am. Ophthalmol. Soc.,* 2006, 29
- **MARIUS ALBOTA.** *Science,* 1998, vol. 281, 1653
- *Advanced Materials,* 2008, 4641
- *J. Phys. Chem. A,* 2011, 105